# EUROPEAN PATENT SPECIFICATION

(11) **EP 3 443 126 B1**
(45) Date of publication and mention of the grant of the patent: **15.11.2023**
(21) Application number: 17783357.1
(22) Date of filing: 17.04.2017
(51) Int. Cl.: C12Q 1/6841, G01N 1/30, G01N 33/53, G01N 33/58

(54) **TISSUE PROFILING USING MULTIPLEXED IMMUNOHISTOCHEMICAL CONSECUTIVE STAINING**
GEWEBEPROFILIERUNG UNTER VERWENDUNG VON MULTIPLEXIERTEN IMMUNHISTOCHEMISCHEN AUFEINANDERFOLGENDEN FÄRBUNGEN
PROFILAGE DE TISSUS PAR COLORATION CONSÉCUTIVE IMMUNOHISTOCHIMIQUE MULTIPLEXÉE

(30) Priority: 15.04.2016 US 201662323172 P
(43) Date of publication of application: 20.02.2019
(73) Proprietor: Icahn School of Medicine at Mount Sinai, New York, NY 10029 (US)
(72) Inventor: REMARK, Romain, 13009 Marseille (FR); MERAD, Miriam, New York, NY 10028 (US); GNJATIC, Sacha, New York, NY 10028 (US)
(74) Representative: Studio Torta S.p.A.
(86) International application number: PCT/US2017/027913
(87) International publication number: WO 2017/181176

(56) References cited:
- WO-A1-2013/015740
- WO-A1-2013/181127
- WO-A1-2014/031068
- US-A1- 2003 017 491
- US-A1- 2005 032 129
- US-A1- 2010 047 825
- US-A1- 2012 021 439
- GIESEN et al.: "Highly Multiplexed Imaging of Tumor Tissues with Subcellular Resolution by Mass Cytometry", Nature Methods, vol. 11, 1 April 2014 (2014-04-01), pages 417-422, XP055186796,
- REMARK et al.: "In-Depth Tissue Profiling Using Multiplexed Immunohistochemical Consecutive Staining on Single Slide", Science Immunology, vol. 1, 14 July 2016 (2016-07-14), pages 1-11, XP055580801, DOI: 10.1126/sciimmunol.aaf6925
- GERDES et al.: "Highly Multiplexed Single- Cell Analysis of Formalin-Fixed, Paraffin-Embedded 'Cancer Tissue", Proceedings of the National Academy of Sciences, vol. 110, 16 July 2013 (2013-07-16), pages 11982-11987, XP055283015,
- REMARK et al.: "Abstract B109: In-depth tissue analysis using multiplexed immunohistochemical consecutive staining on single slide", Cancer Immunology Research, vol. 4, no. Iss. 1, 3 January 2016 (2016-01-03), page B109, XP009513616,
- STACK et al.: "Multiplexed Immunohistochemistry, Imaging, and Quantitation: a Review, with an Assessment of Tyramide Signal Amplification, Multispectral Imaging and Multiplex Analysis", Methods, vol. 70, 30 November 2014 (2014-11-30), pages 46-58, XP055273702,

## Description

### FIELD OF THE INVENTION

The present invention relates to methods for sequential multidimensional immunohistochemical analyses of tissues.

### BACKGROUND

Despite remarkable recent achievements of immunotherapy strategies in cancer treatment, clinical responses remain limited to subsets of patients. Novel predictive markers of disease course and response to immunotherapy are urgently needed. Recent results have revealed the potential predictive value of immune cell phenotype and spatial distribution at the tumor site, prompting the need for multidimensional immunohistochemical analyses of tumor tissues. The visualization and quantification of different immune cellular subsets requires the use of complex phenotypic marker combinations. A major limitation for such high dimensional analyses is tumor tissue availability. Most clinical pathology laboratories use chromogenic immunohistochemistry (IHC) on commonly accessible formalin-fixed paraffin-embedded (FFPE) tissues and stain for no more than two markers per tissue slide (9). Several commercial multiplexed immunostaining methods have been developed to allow high dimensional analysis of complex immune cell populations but most of these methods have inherent limitations, including the use of proprietary fluorescent probes that stray from accepted standards in pathology, the dependency on frozen material, the associated tissue destruction, and the requirement of costly equipment, materials, and reagents (18-22).

In cancer, evidence of immunocompetence at the tumor site has been associated with improved outcome of patients with various tumor types (4,5) and several studies established that high lymphocyte infiltration in tumors is prognostic of progression-free or overall survival (6, 7). A landmark study in colon cancer lesions demonstrated that the density of two lymphocyte populations (CD3/CD8, CD3/CD45RO, or CD8/CD45RO) in two tumor regions (center and invasive margin) is a better predictor of survival than the TNM stage (6, 8). As a result, pathologists around the world are developing a task force to validate the use of CD3/CD8 infiltration named "Immunoscore", to complement standard staging in routine clinical cancer settings (9). The sole measurement of CD3/CD8 cell infiltration in tumors, although useful in colorectal cancer is not predictive in all solid tumors where other immune cell populations might be associated with favorable clinical outcome (10-12), revealing the critical need for a more comprehensive analysis of the immune microenvironment of tumor tissues.

US 2012/021439 discloses a method called Sequential IMmunoPeroxidase Labeling and Erasing (SIMPLE) that enables the simultaneous visualization of at least five markers within a single tissue section. Utilizing the alcohol-soluble peroxidase substrate 3-amino-9-ethylcarbazole (AEC), combined with a rapid non-destructive method for antibody-antigen dissociation, the method allows to erase the results of a single immunohistochemical stain while preserving tissue antigenicity for repeated rounds of labeling. The method also allows to visualize multiple antigens simultaneously.

### SUMMARY

The present invention relates to a method as defined in claim 1. Specific embodiments of the invention are outlined in the dependent claims.

### BRIEF DESCRIPTION OF THE DRAWINGS

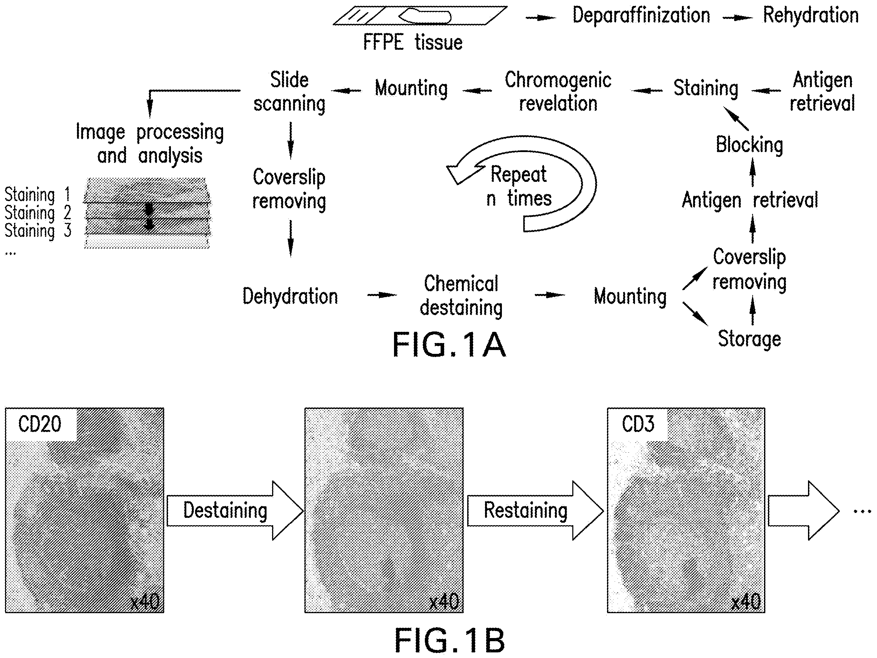

**Figures 1A-B** **are a flow chart and exemplary images of immunohistochemistry stains exemplifying the Multiplexed Immunohistochemical Consecutive Staining on Single Slide (MICSSS) protocol.** **Fig. 1A** is a flow chart showing the MICSSS protocol using five µm FFPE tissue sections incubated with primary Abs followed by biotinylated secondary Abs, streptavidin-horse radish peroxidase and AEC. Stained tissue sections were counterstained, mounted and scanned. After each scanning procedure, the slide coverslip was removed and AEC chromogen was dissolved. Tissue sections underwent antigen retrieval and then were incubated in a blocking buffer prior to the initiation of a new staining cycle. **Fig. 1B** are images of immunohistochemistry stains of five µm FFPE gut section obtained from an ulcerative colitis patient stained with anti-CD20 Ab, destained and stained with anti-CD3 Ab according to the MICSSS workflow/protocol. The exact same tissue can be sequentially stained multiple times using the MICSSS method, conserving tissue sample. Original magnification: x40.

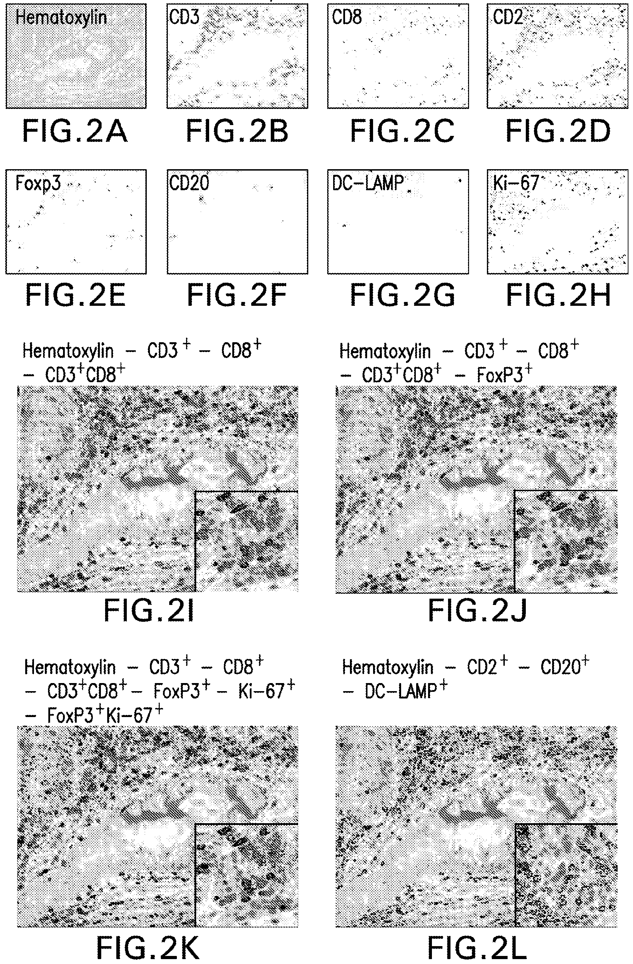

**Figures 2A-L** **are IHC stains generated using MICSSS to characterize the tumor immune microenvironment.** Colorectal cancer tissue section was sequentially stained eight times and scanned for hematoxylin, CD3, CD8, CD2, FoxP3, CD20, DC-LAMP and Ki-67. Bright field images were inverted and RGB channel splitting was performed. Upper panels show single staining for each individual staining. Some selected images were merged and pseudo-colors attributed to each marker (lower panels). Immune cells were mostly localized in the stroma surrounding the tumor islets. Right inserts show magnifications of single, double (e.g. CD3+ CD8+ or CD3+ FoxP3+) or triple (e.g. CD3+ FoxP3+ Ki-67+) positive cells allowing an accurate determination of cell phenotype and state. Original magnification: x200 and x300.

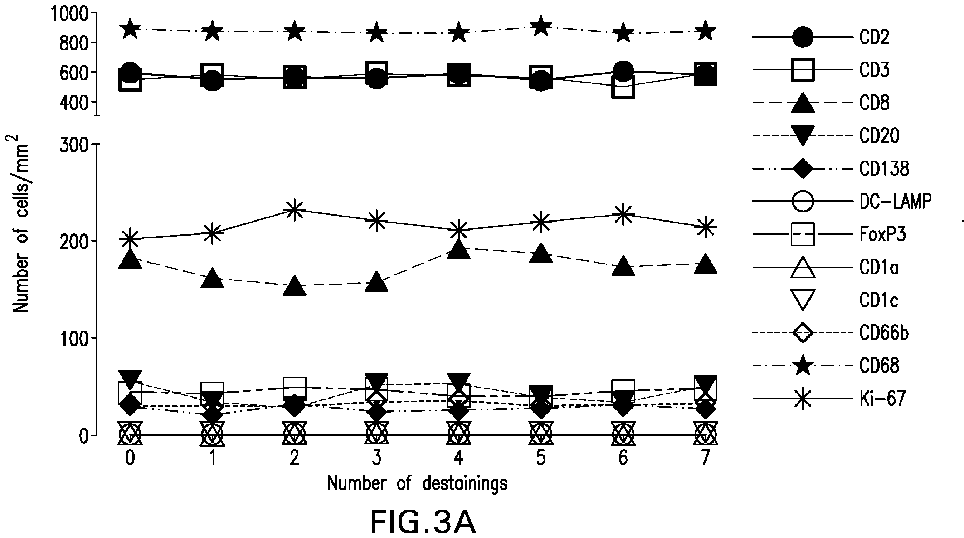

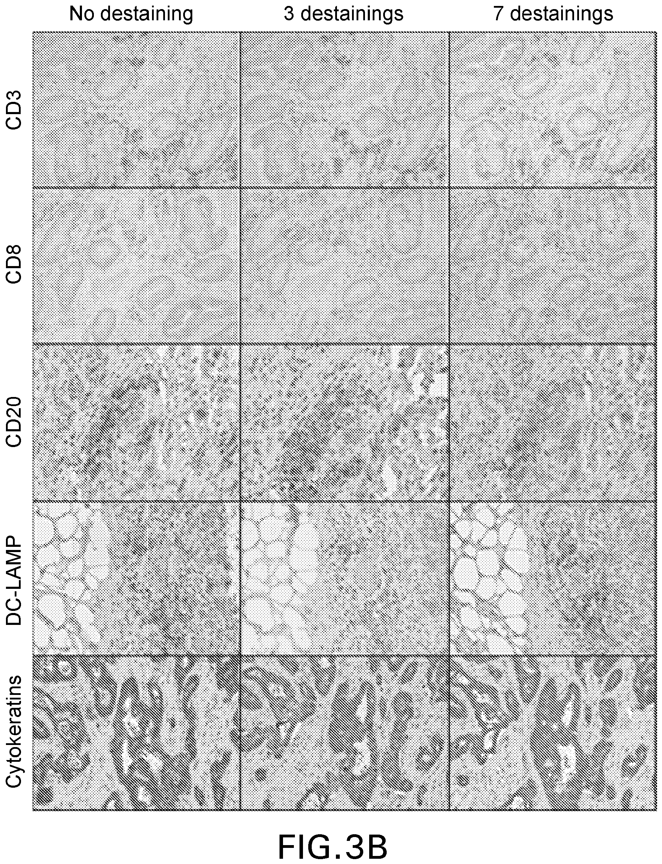

**Figures 3A-B** **and graphs and staining showing that MICSSS surprisingly does not alter tissue antigenicity.** **Fig. 3A** shows a series of 12 adjacent 5 µm FFPE sections that were obtained from a colorectal tumor tissue. Each slide was stained with a panel of Abs using the MICSSS workflow, but in a different Ab sequence order for each slide. Line graph shows the densities of tumor associated immune cells positive for CD1a, CD1c, CD2, CD3, CD8, CD20, CD66b, CD68, CD138, FoxP3, DC-LAMP and Ki-67 positive cells whether each marker was stained prior or after 1 to 7 destaining cycles. **Fig. 3B** are representative staining images revealing 23 similar staining intensity whether the marker was stained prior or after several staining/destaining cycles. Original magnification: x66.

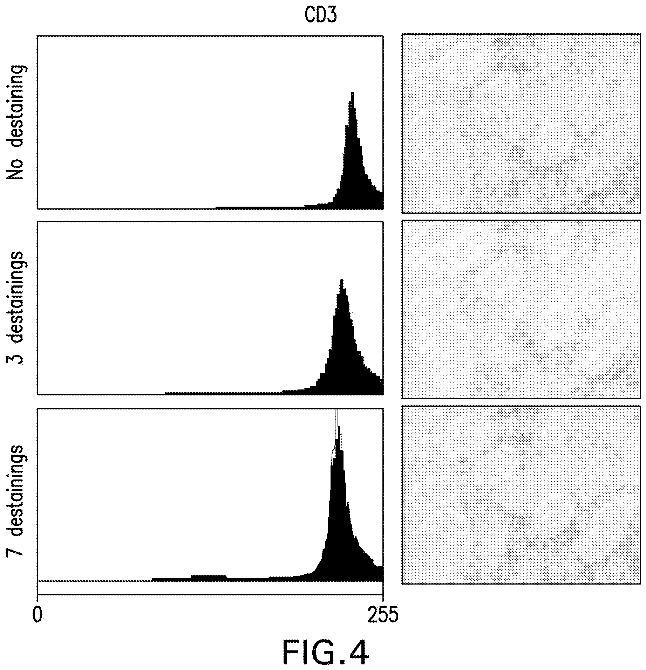

**Figure 4** **includes histograms demonstrating that MICSSS does not alter the signal intensity.** Serially 5 µm tissue sections were stained with CD3 following the MICSSS workflow and the intensity of the signal was assessed after several destaining. After color deconvolution, the intensity histograms were drawn, revealing similar signal intensities whether the staining was performed prior or after several cycles of destaining/restaining cycles.

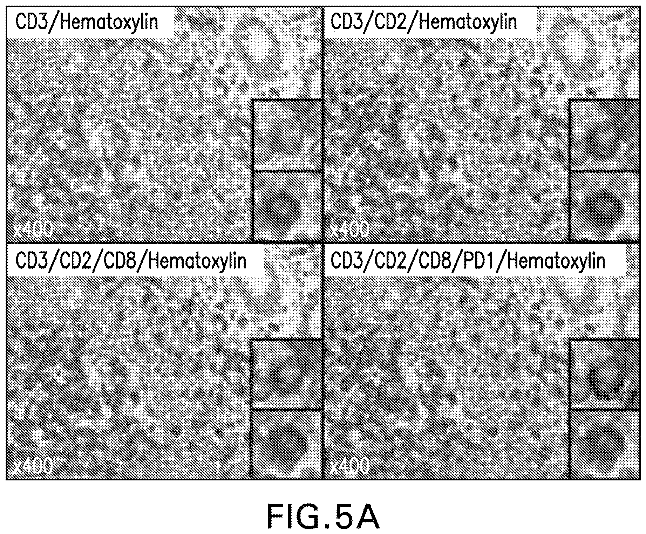

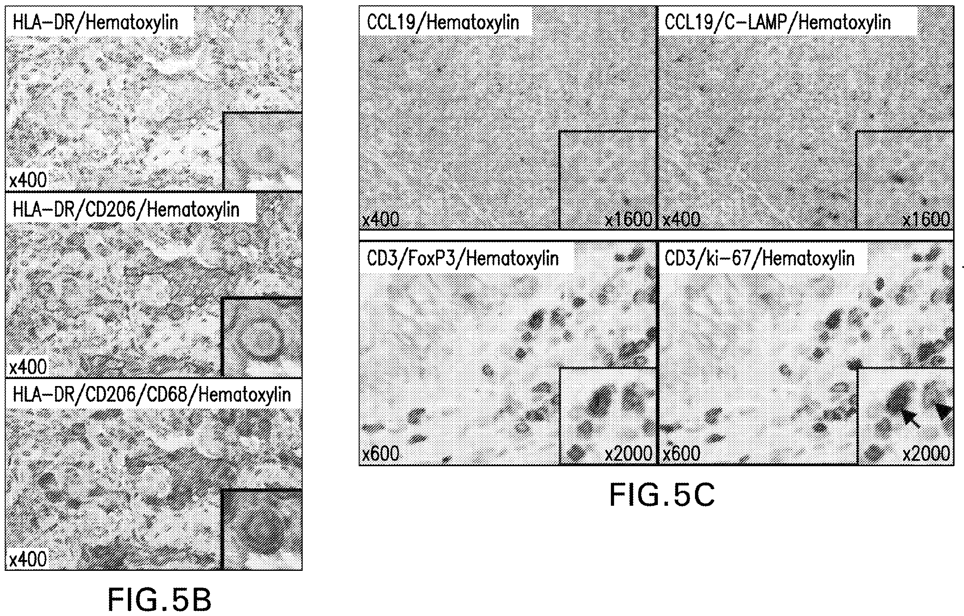

**Figures 5A-C** **includes stains demonstrating visualization of multiple antigens on single cells using MICSSS.** **Fig. 5A** includes panels of images showing the co-expression of CD3, CD2, CD8 and PD-1 markers on the cell surface of colorectal cancer-associated T cells. **Fig. 5B** **shows** co-expression of HLA-DR, CD206 and CD68 on lung tumor-associated macrophages and **Fig. 5C** **shows** co-expression of cytoplasmic CCL19 and DC-LAMP and nuclear FoxP3 and Ki-67 on CD3+ T cells in tonsil and colorectal cancer tissues section, respectively. Black arrow shows FoxP3/Ki-67 double positive cell and black head arrow shows FoxP3+ Ki-67- cell. Original magnification: x400, x600, x1600 and x2000.

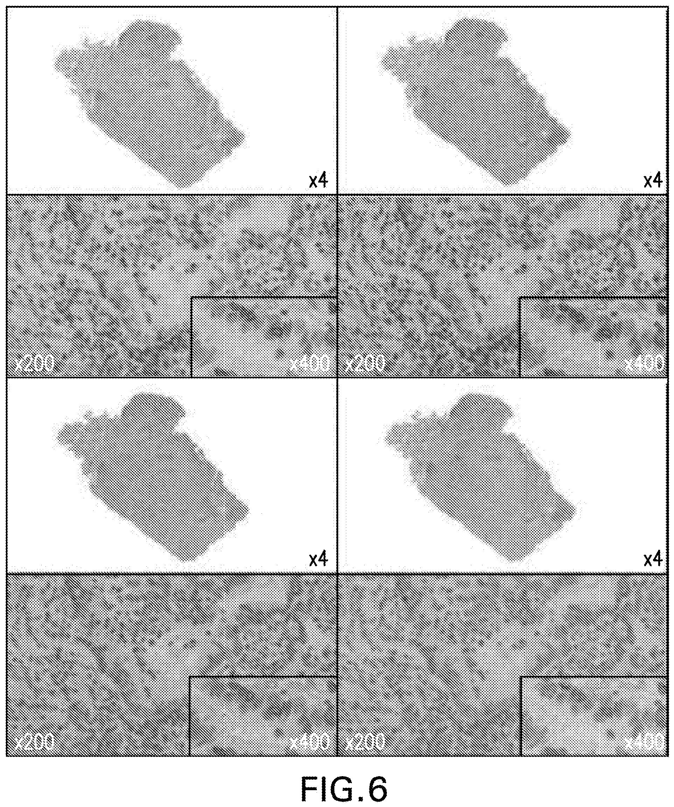

**Figure 6** **includes panels showing sequential CD3 staining using MICSSS.** A 5 µm FFPE NSCLC tissue section was repeatedly stained, destained, and restained with the same anti-CD3 Ab. Images show identical number and distribution of tumor infiltrating CD3+ T cells when the tissue section was stained with anti-CD3 Ab (upper left panel), destained and restained for CD3 for a second time (upper right panel), a third time (lower left panel) or a fourth time (lower right panel). Original magnification: x4, x200 and x400.

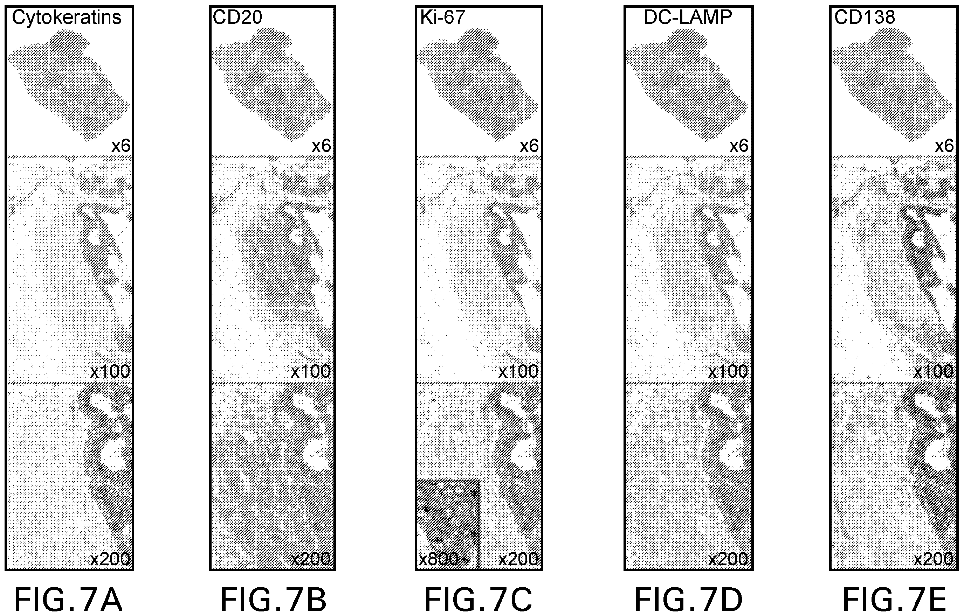

**Figures 7A-E** **are stains showing that MICSSS can selectively remove one chromogen-stained marker while preserving a fixed diagnostic marker.** Lung adenocarcinoma tissue section was permanently stained with anti-cytokeratins Abs (clones AE1/AE3) revealed by DAB (brown, dark staining indicated in Fig. 7A) and sequentially stained and destained with anti-CD20, -Ki-67, -DC-LAMP and -CD138 Abs and revealed by AEC in red (additional staining shown in Figs. 7B-E). The cytokeratin staining was kept as a reference along the staining process, as the destaining process, which selectively removed only the AEC stain, did not affect it. Original magnification: x6, x100, x200 and x800.

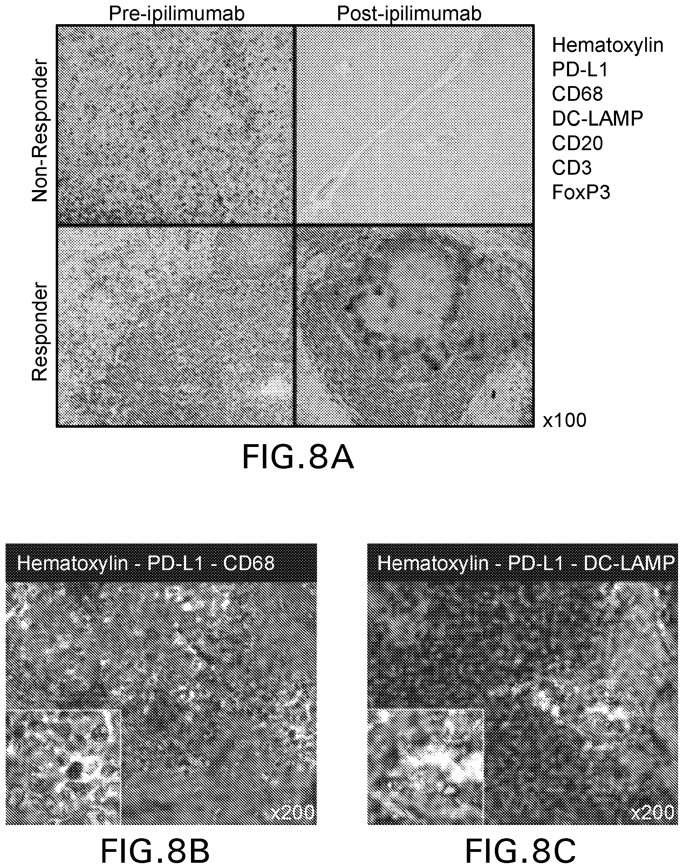

**Figures 8A****-C: are stains showing that MICSSS can monitor tumor response to immunotherapy regimens.** **Fig. 8A** shows five µm FFPE melanoma tissue sections isolated prior and after treatment with ipilimumab from one responder and one non-responder patient were stained with the MICSSS method. Each tissue section was stained sequentially with hematoxylin and anti-PD-L1, -CD68, -DC-LAMP, -CD20, -CD3 and -FoxP3 Abs and images were overlaid. **Figs. 8B-C** are images that show the expression of PD-L1 by either CD68+ macrophages **(****Fig. 8B****)** and DC-LAMP+ mature DCs **(****Fig. 8C****)** in a responder patient. Original magnification: x100 (Fig. 5A) and x200 **(****Fig. 5B**,C).

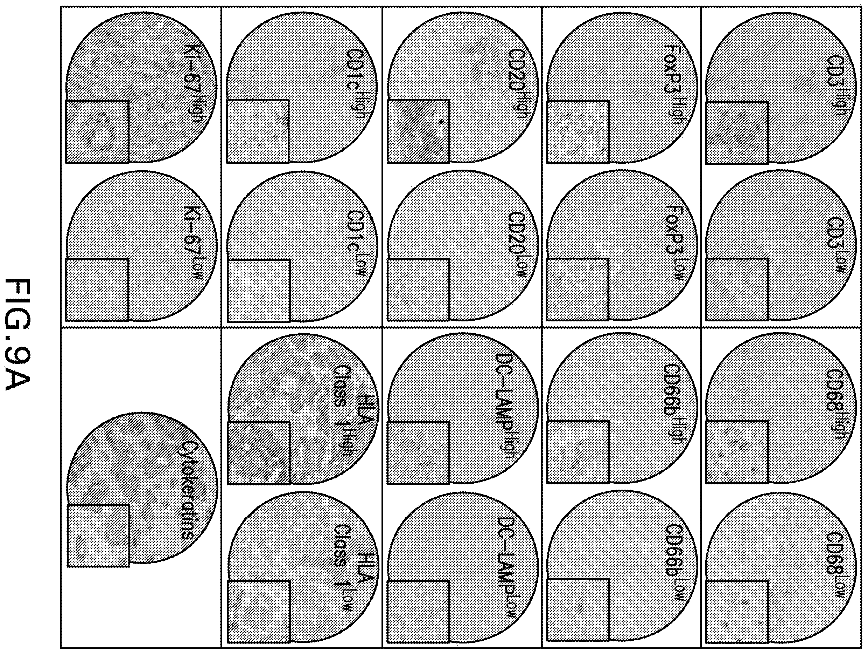

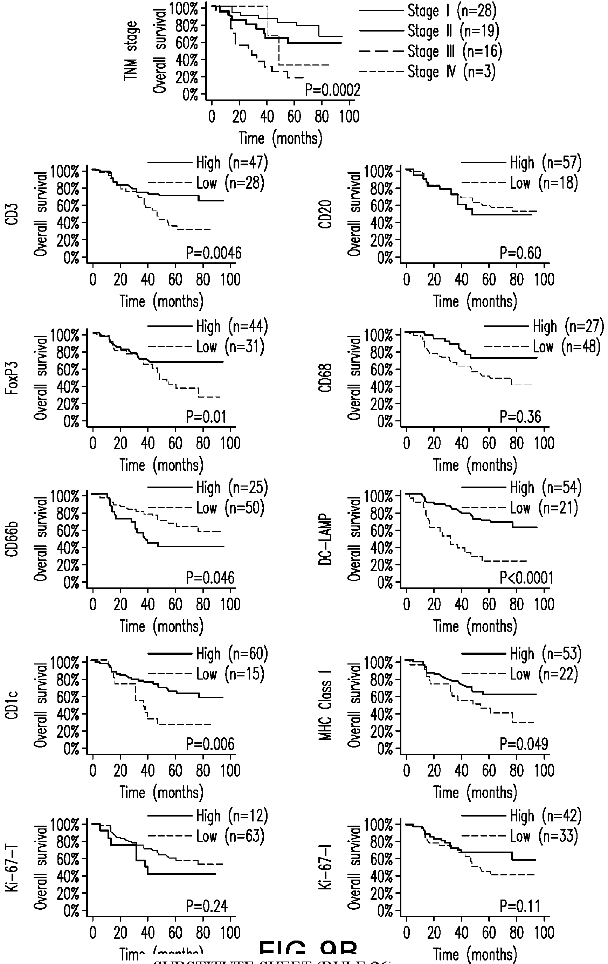

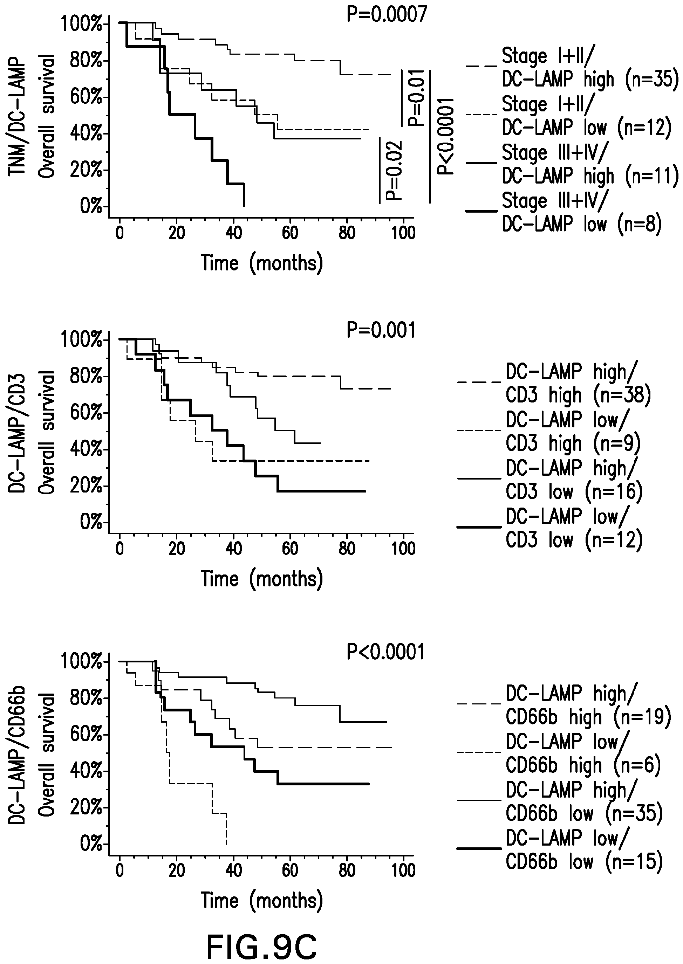

**Figures 9A-C** **are stains and graphs showing that MICSSS can identify novel immune prognostic markers in cancer patients.** **Fig. 9A** **shows** representative images of different biopsy sections obtained from NSCLC tissue microarray sequentially stained with anti-CD3, -CD20, -FoxP3, -CD68, -CD66b, -DCLAMP, -CD1c, -MHC class I, -Ki-67 and - cytokeratins Abs. Original magnification: x40 and x200 (right inserts). **Fig. 9B** shows Kaplan-Meier curves that illustrate the duration of overall survival according to the TNM stage and the densities of CD3+, CD20+, FoxP3+, CD68+ CD66b+, DC-LAMP+, CD1c+, Ki-67+ and MHC class I+ cells. For the density curves, solid lines represent high cell densities (or high expression) and dashed lines, low densities (or low expression). **Fig. 9C** are Kaplan-Meier curves illustrating the duration of overall survival according to the combined analysis of TNM stage and immune cell densities (CD3+, DC-LAMP+ and CD66b+).

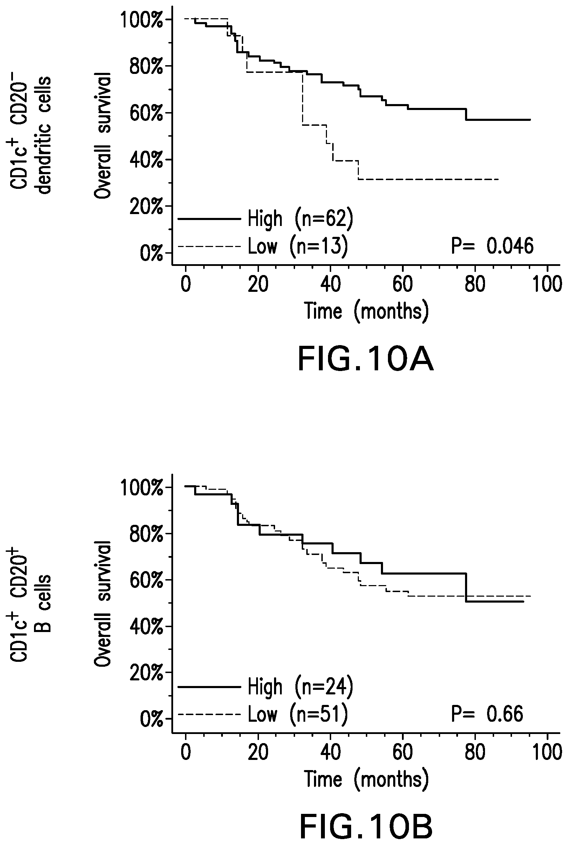

**Figure 10A-B** **are graphs showing the prognostic value of CD1c positive cells.** **Fig. 10A** are Kaplan-Meier curves illustrate the duration of overall survival according to the density of NSCLC-infiltrating CD1c+ CD20- dendritic cells and CD1c+ CD20+ B cells **(****Fig. 10B****).** Solid lines represent high cell densities and dashed lines represent low densities.

### DETAILED DESCRIPTION

The immune system is formed by an incredibly diverse network of cells derived from the myeloid and lymphoid hematopoietic lineages that cooperate to sense and respond to tissue injury signals. Recent studies have revealed that immune cell types initially believed to represent a single lineage in fact consist of different subpopulations with distinct functions (1) and the nature of the responding immune cells and their spatial organization within organs control the development of effective immune responses (2, 3). However, a lack of solutions to characterize this complexity at the tissue site hampers the ability to perform comprehensive *in situ* analyses of ongoing immune responses and to decipher mechanisms at play.

In addition to the powerful prognostic value of tumor-associated immune cells, recent studies have established that antibody (Ab)-mediated blockade of immune checkpoint receptors on T cells, or their ligands on antigen presenting cells such as dendritic cells (DCs) or macrophages, can lead to significant clinical responses in a subset of Patients (13). Three checkpoint inhibitors have been actively explored clinically, including Abs to the checkpoint receptor cytotoxic lymphocyte antigen 4 (CTLA-4), programmed cell death 1 (PD-1) and to the checkpoint ligand PD-L1 (programmed death-ligand 1)(14). Analysis of tumor lesions treated with checkpoint blockade revealed that a pre-existing high density of CD8+ T cells in the center and invasive margin of the tumor mass along with expression of PD-L1 on infiltrating immune cells or tumor cells correlates with increased tumor response to anti-PD-1 and anti-PD-L1 Abs (15, 16). The ability to perform longitudinal high-dimensional analysis of tumor lesions using routine tissue on a single slide would be extremely useful for immune monitoring of cancer patients (17) undergoing treatment.

To address the clinical need for high dimensional analysis of tissue lesions in clinical pathology, a new multiplexed chromogen-based IHC staining assay independent of proprietary equipment has been developed that has the added benefit of readily being able to be integrated into standard clinical pathology settings. This new technique, named Multiplexed Immunohistochemical Consecutive Staining on Single Slide (MICSSS) is based on the labile nature of some chromogens and can be performed on any FFPE tissue using iterative cycles of staining, image scanning, and destaining of chromogenic substrate. The MICSSS method can easily be implemented to most existing staining protocols without increasing risk of antibody cross-reactivity, thus retaining previously established Ab specificity and sensitivity parameters. For example, MICSSS can be performed following *in situ* hybridization (FISH or CISH) using DNA or RNA probes.

The MICSSS method can characterize a large panel of parameters on one single tissue section, including co-localization of markers on single cells while preserving tissue antigenicity and architecture. Because of the use of chromogen, MICSSS is not limited by photo-bleaching or autofluorescence, and allows prolonged slide storage for future use as new markers become available. Finally, a novel automated digital landscaping software based on deep learning was designed, developed, and applied to this multiplexed IHC method, thus facilitating the ability to automatically map and analyze the complexity of the tumor microenvironment (TME). The results described herein illustrate the MICSSS workflow and its clinical potential in numerous fields, including to identify prognostic and predictive factors of disease course, or predictive biomarkers of response to immunotherapy.

Embodiments of the present invention relate to a sample-sparing, highly multiplexed immunohistochemistry techniques based on iterative cycles of tagging, image scanning, and destaining of chromogenic substrate on a single slide. These methods, in combination with automated digital landscaping techniques, provide a broad opportunity for high-dimensional immunohistochemical analyses by capturing the complexity of the immunome *in situ* using readily available pathology standards. Applications of the MICSSS method extend beyond predicting responsiveness to cancer treatments, but also apply to screening and validation of comprehensive panels of tissue-based prognostic and predictive markers, as well as in-depth *in situ* monitoring of therapies, and to identification of novel disease targets.

### ABBREVIATIONS

3-amino-9-ethylcarbazole: (AEC);
3,3'-Diaminobenzidine: (DAB);
Formalin-fixed paraffin-embedded tissue: FFPE tissue;
IHC: Immunohistochemistry;
MICSSS: Multiplexed Immunohistochemical Consecutive Staining on Single Slide;
RGB: red green blue;
TME: tumor microenvironment.

### DEFINITIONS

The singular forms "a" "an" and "the" include plural referents unless the context clearly dictates otherwise.

"Affinity" is defined as the strength of the binding interaction of two molecules, such as an antigen and its antibody, which is defined for antibodies and other molecules with more than one binding site as the strength of binding of the ligand at one specified binding site. Although the noncovalent attachment of a ligand to antibody is typically not as strong as a covalent attachment, "High affinity" is for a ligand that binds to an antibody having an affinity constant (Kₐ) of greater than 10⁴ M⁻¹, typically 10⁵-10¹¹ M⁻¹; as determined by inhibition ELISA or an equivalent affinity determined by comparable techniques such as, for example, Scatchard plots or using Kd/dissociation constant, which is the reciprocal of the Kₐ, etc.

"Antibody" is defined as a protein of the immunoglobulin (Ig) superfamily that binds noncovalently to certain substances (e.g. antigens and immunogens) to form an antibody-antigen complex, including but not limited to antibodies produced by hybridoma cell lines, by immunization to elicit a polyclonal antibody response, by chemical synthesis, and by recombinant host cells that have been transformed with an expression vector that encodes the antibody. In humans, the immunoglobulin antibodies are classified as IgA, IgD, IgE, IgG, and IgM and members of each class are said to have the same isotype. Human IgA and IgG isotypes are further subdivided into subtypes IgAi, and IgA₂, and IgG₁, IgG₂, IgG₃, and IgG₄. Mice have generally the same isotypes as humans, but the IgG isotype is subdivided into IgG₁, IgG₂ₐ, IgG_{2b}, and IgG₃ subtypes. Thus, it will be understood that the term "antibody" as used herein includes within its scope (a) any of the various classes or sub-classes of immunoglobulin, e.g., IgG, IgM, IgE derived from any of the animals conventionally used and (b) polyclonal and monoclonal antibodies, such as murine, chimeric, or humanized antibodies. Antibody molecules have regions of amino acid sequences that can act as an antigenic determinant, e.g. the Fc region, the kappa light chain, the lambda light chain, the hinge region, etc. An antibody that is generated against a selected region is designated anti-(region), e.g. anti-Fc, anti-kappa light chain, anti-lambda light chain, etc. An antibody is typically generated against an antigen by immunizing an organism with a macromolecule to initiate lymphocyte activation to express the immunoglobulin protein.

The term antibody, as used herein, also covers any polypeptide, antibody fragment, or protein having a binding domain that is, or is homologous to, an antibody binding domain, including, without limitation, single-chain Fv molecules (scFv), wherein a VH domain and a VL domain are linked by a peptide linker that allows the two domains to associate to form an antigen binding site (Bird et al., Science 242, 423 (1988) and Huston et al., Proc. Natl. Acad. Sci. USA 85, 5879 (1988)). These can be derived from natural sources, or they may be partly or wholly synthetically produced.

"Antibody fragments" is defined as fragments of antibodies that retain the principal selective binding characteristics of the whole antibody. Particular fragments are well-known in the art, for example, Fab, Fab', and F(ab')₂, which are obtained by digestion with various proteases and which lack the Fc fragment of an intact antibody or the so-called "half-molecule" fragments obtained by reductive cleavage of the disulfide bonds connecting the heavy chain components in the intact antibody. Such fragments also include isolated fragments consisting of the light-chain-variable region, "Fv" fragments consisting of the variable regions of the heavy and light chains, and recombinant single chain polypeptide molecules in which light and heavy variable regions are connected by a peptide linker. Other examples of binding fragments include (i) the Fd fragment, consisting of the VH and CH1 domains; (ii) the dAb fragment (Ward, et al., Nature 341, 544 (1989)), which consists of a VH domain; (iii) isolated CDR regions; and (iv) single-chain Fv molecules (scFv) described above. In addition, arbitrary fragments can be made using recombinant technology that retains antigen-recognition characteristics.

"Antigen" is defined as a molecule that induces, or is capable of inducing, the formation of an antibody or to which an antibody binds selectively, including but not limited to a biological material. Antigen also refers to "immunogen". An antibody binds selectively to an antigen when there is a relative lack of cross-reactivity with or interference by other substances present.

"Biological sample" or "Biological material" is defined as a sample retrieved from an animal, mammals and human beings in particular. The sample may be of a healthy tissue, disease tissue or tissue suspected of being disease tissue. The sample may be a biopsy taken, for example, during a surgical procedure. The sample may be collected via means of fine needle aspiration, scraping or washing a cavity to collects cells or tissue therefrom. The sample may be of a tumor e.g., solid and hematopoietic tumors as well as of neighboring healthy tissue. The sample may be a smear of individual cells or a tissue section. Typically, the sample comprises tissue, cell or cells, cell extracts, cell homogenates, purified or reconstituted proteins, recombinant proteins, bodily and other biological fluids, viruses or viral particles, prions, subcellular components, or synthesized proteins. Possible sources of cellular material used to prepare the sample include without limitation plants, animals, fungi, protists, bacteria, archae, or cell lines derived from such organisms.

"Complex" is defined as two or more molecules held together by noncovalent bonding, which are typically noncovalent combinations of biomolecules such as a protein complexed with another protein. In contrast, a protein is covalently labeled with a substance when there is a covalent chemical bond between the substance and the protein.

"Detectably distinct" is defined as the signal being distinguishable or separable by a physical property either by observation or instrumentally. For example, but not limitation, a fluorophore is readily distinguishable, either by spectral characteristics or by fluorescence intensity, lifetime, polarization or photo-bleaching rate from another fluorophore in the sample, as well as from additional materials that are optionally present.

"Directly detectable" is defined to mean that the presence of a material or the signal generated from the material is immediately detectable by observation, instrumentation, or film without requiring chemical modifications.

"Immunoconjugates" is defined to mean that labeling proteins, where instead of a detectable label being attached to the protein, a therapeutic agent or drug is attached. The term immunoconjugate is used interchangeably with drug-labeled protein.

"Monovalent antibody fragment" is defined as an antibody fragment that has only one antigen-binding site. Examples of monovalent antibody fragments include, but are not limited to, Fab fragments (no hinge region), Fab' fragments (monovalent fragments that contain a heavy chain hinge region), and single-chain fragment variable (ScFv) proteins.

"Multiplex identification" refers to the simultaneous identification of one or more targets in a single mixture. For example, a two-plex amplification refers to the simultaneous identification, in a single reaction mixture, of two different targets.

"Selectively binds" is defined as the situation in which one member of a specific intra or inter species binding pair will not show any significant binding to molecules other than its specific intra- or inter-species binding partner (e.g., an affinity of about 100-fold less), i.e. minimal cross-reactivity.

### Detection Methods

In various aspects the disclosure provides methods of detecting a target in a biological sample. Targets are detected by contacting a biological sample with a target detection reagent, e.g., an antibody or fragment thereof and a labeling reagent. Targets are detected by the presence or absence of the detection reagent-labeling reagent complex. Preferably, the biological sample is contacted with the target detection reagent and the labeling reagent sequentially. For example, the biological sample is incubated with the detection reagent under conditions that allow a complex between the detection reagent and target to form. After complex formation the biological sample is optionally washed one or more times to remove unbound detection reagent. The biological sample is further contacted with a labeling reagent that specifically binds the detection reagent that is bound to the target. The biological sample is optionally washed one or more times to remove unbound labeling reagent. The presence or absence of the target in the biological sample is then determined by detecting the labeling reagent. Alternatively, the biological sample is contacted with the target detection reagent and the labeling reagent concurrently.

The invention provides for the sequential detection of multiple antigen targets in a sample as detailed in the appended claims. Multiple targets include the discrete epitope that the target-binding antibody has affinity for as well as molecules or structures that the epitope is bound to. Thus, multiple target identification includes phenotyping of cells based on the concentration of the same cell surface marker on different cells. In this way multiple target identification is not limited to the discrete epitope that the target binding antibody binds, although this is clearly a way that multiple targets can be identified, i.e. based on the affinity of the target-binding antibody.

The sample is defined to include any material that may contain a target to which an antibody has affinity. Typically the sample is biological in origin and comprises tissue, cell or a population of cells, cell extracts, cell homogenates, purified or reconstituted proteins, recombinant proteins, bodily and other biological fluids, viruses or viral particles, prions, subcellular components, or synthesized proteins. The sample is a biological fluid such as whole blood, plasma, serum, nasal secretions, sputum, saliva, urine, sweat, transdermal exudates, or cerebrospinal fluid. Alternatively, the sample may be whole organs, tissue or cells from an animal. Examples of sources of such samples include muscle, eye, skin, gonads, lymph nodes, heart, brain, lung, liver, kidney, spleen, solid tumors, macrophages, or mesothelium. The sample is prepared in a way that makes the target, which is determined by the end user, in the sample accessible to the immuno-labeled complexes. Typically, the samples used in the invention are comprised of tissue or cells. Preferably, the tissue or cells to be assayed will be obtained by surgical procedures, e.g., biopsy. The tissue or cells are fixed, or frozen to permit histological sectioning. In situ detection is used to determine the presence of a particular target and to determine the distribution of the target in the examined tissue. General techniques of in situ detection are well known to those of ordinary skill. See, for example, Ponder, "Cell Marking Techniques and Their Application," in Mammalian Development: A Practical Approach, Monk (ed.), 115 (1987). Treatments that permeabilize the plasma membrane, such as electroporation, shock treatments, or high extracellular ATP, can be used to introduce reagents into cells.

The target is any compound of biological or synthetic origin that is present as a molecule or as a group of molecules. Typically, the target is a biological material or antigenic determinant. The chemical identity of the target antigen may be known or unknown. Biological materials include, but are not limited to, antibodies, amino acids, proteins, peptides, polypeptides, enzymes, enzyme substrates, hormones, lymphokines, metabolites, antigens, haptens, lectins, avidin, streptavidin, toxins, poisons, environmental pollutants, carbohydrates, oligosaccharides, polysaccharides, glycoproteins, glycolipids, nucleotides, oligonucleotides, nucleic acids and derivatized nucleic acids (including deoxyribo- and ribonucleic acids and peptide nucleic acids), DNA and RNA fragments and derivatized fragments (including single and multi-stranded fragments), natural and synthetic drugs, receptors, virus particles, bacterial particles, virus components, biological cells, cellular components (including cellular membranes and organelles), natural and synthetic lipid vesicles, and polymer membranes. Typically the target material is present as a component or contaminant of a sample taken from a biological or environmental system.

The target can include a transmembrane marker. Alternatively, the target is an intracellular or a nuclear antigen. Intracellular antigen include, for example, alpha-fetoprotein (AFP), human chorionic gonadotropin (HCG), colon-specific antigen-p (CSAp), prostatic acid phosphatase, pancreatic oncofetal antigen, placental alkaline phosphatase, parathormone, calcitonin, tissue polypeptide antigen, galactosyl transferase-II (GT-II), gp-52 viral-associated antigen, ovarian cystadenocarcinoma-associated antigen (OCAA), ovarian tumor-specific antigen (OCA), cervical cancer antigens (CA-58, CCA, TA-4), basic fetoprotein (BFP), terminal deoxynucleotidyl transferase (TdT), cytoplasmic melanoma-associated antigens, human astrocytoma-associated antigen (HAAA), common glioma antigen (CGA), glioembryonic antigen (GEA), glial fibrillary acidic protein (GFA), common meningioma antigen (CMA), pMTOR, pAKT, PSMA, prostate specific antigen (PSA), x-methylacyl-CoA racemase (AMACR), vascular endothelial growth factor (VEGF), and tumor angiogenesis factor (TAF). Nuclear antigens include for example, PTEN, Ki67, Cyclin D1, EZH2, p53, IGFBP2, p-STAT-3. Other targets include those listed on Tables 1-3 below.

The detection reagent is a compound that is capable of specifically binding to the target of interest. The detection reagent is selected based on the desired target. The detection reagent is for example a polypeptide such as a target specific antibody or fragment thereof. As used herein, the term "antibody" refers to immunoglobulin molecules and immunologically active portions of immunoglobulin (Ig) molecules, i.e., molecules that contain an antigen binding site that specifically binds (immunoreacts with) an antigen. Such antibodies include, polyclonal, monoclonal, chimeric, single chain, F_{ab}, F_{ab'} and F_{(ab')2} fragments, and an F_{ab} expression library. By "specifically bind" or "immunoreacts with" is meant that the antibody reacts with one or more antigenic determinants of the desired antigen and does not react (i.e., bind) with other polypeptides or binds at much lower affinity (K_{d}>10⁻⁶) with other polypeptides.

Monoclonal antibodies are particularly advantageous in practicing the methods of the present invention. Generally, monoclonal antibodies are more sensitive and specific than polyclonal antibodies. In addition, unlike polyclonal antibodies, which depend upon the longevity of the animal producing the antibody, the supply of monoclonal antibodies is indefinite. Polyclonal antibodies however, are useful when it is necessary to use antibodies with multiple isotypes, as generally most monoclonal antibodies are of the IgG1 subclass.

As used herein, the term "epitope" includes any protein determinant capable of specific binding to an immunoglobulin, an scFv, or a T-cell receptor. The term "epitope" includes any protein determinant capable of specific binding to an immunoglobulin or T-cell receptor. Epitopic determinants usually consist of chemically active surface groupings of molecules such as amino acids or sugar side chains and usually have specific three-dimensional structural characteristics, as well as specific charge characteristics.

As used herein, the terms "immunological binding," and "immunological binding properties" refer to the non-covalent interactions of the type that occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides are quantified using methods well known in the art. One such method entails measuring the rates of antigen-binding site/antigen complex formation and dissociation, wherein those rates depend on the concentrations of the complex partners, the affinity of the interaction, and geometric parameters that equally influence the rate in both directions. Thus, both the "on rate constant" (Kₒₙ) and the "off rate constant" (K_{off}) can be determined by calculation of the concentrations and the actual rates of association and dissociation. (See Nature 361:186-87 (1993)). The ratio of K_{off}/Kₒₙ enables the cancellation of all parameters not related to affinity, and is equal to the dissociation constant K_{d}. (See, generally, Davies et al. (1990) Annual Rev Biochem 59:439-473).

The labeling reagent contains an antibody binding moiety and a detection moiety. The antibody binding moiety and the detection moiety are covalently linked. Alternatively, the antibody binding moiety and the detection moiety are non-covalently linked.

The antibody binding moiety bind selectively and with high affinity to a selected region of the detection reagent, e.g., the target-binding antibody. The binding region for the antibody binding moiety may be a selected peptide linker (including the J region), light chain or heavy chain of the target-binding antibody; preferably the labeling protein binds the Fc region of the target-binding antibody.

The antibody binding moiety is an antibody or fragment thereof, such as, but not limited to, anti-Fc, an anti-Fc isotype, anti-J chain, anti-kappa light chain, anti-lambda light chain, or a single-chain fragment variable protein. Preferably, the antibody binding moiety is monovalent. Alternatively, the antibody binding moiety is a non-antibody peptide or protein, such as, for example but not limited to, soluble Fc receptor, protein G, protein A, protein L, lectins, or a fragment thereof. Optionally, the non-antibody protein or peptide is coupled with albumin such as human and bovine serum albumins or ovalbumin.

Typically, the antibody binding moiety is a Fab fragment specific to the Fc portion of the target-binding antibody or to an isotype of the Fc portion of the target-binding antibody. The monovalent Fab fragments are produced from either murine monoclonal antibodies or polyclonal antibodies generated in a variety of animals, for example but not limited to, rabbit or goat. These fragments can be generated from any isotype such as murine IgM, IgG₁, IgG₂ₐ, IgG_{2b} or IgG₃.

The detection moiety, i.e., label, is any substance used to facilitate identification and/or quantitation of a target. Detection moieties are directly observed or measured or indirectly observed or measured. Detection moieties include, but are not limited to, radiolabels that can be measured with radiation-counting devices; pigments, dyes or other chromogens that can be visually observed or measured with a spectrophotometer; spin labels that can be measured with a spin label analyzer; and fluorescent moieties, where the output signal is generated by the excitation of a suitable molecular adduct and that can be visualized by excitation with light that is absorbed by the dye or can be measured with standard fluorometers or imaging systems, for example. The detection moiety can be a luminescent substance such as a phosphor or fluorogen; a bioluminescent substance; a chemiluminescent substance, where the output signal is generated by chemical modification of the signal compound; a metal-containing substance; or an enzyme, where there occurs an enzyme-dependent secondary generation of signal, such as the formation of a colored product from a colorless substrate. The detection moiety may also take the form of a chemical or biochemical, or an inert particle, including but not limited to colloidal gold, microspheres, quantum dots, or inorganic crystals such as nanocrystals or phosphors (see, e.g., Beverloo, et al., Anal. Biochem. 203, 326-34 (1992)). The term detection moiety can also refer to a "tag" or hapten that can bind selectively to a labeled molecule such that the labeled molecule, when added subsequently, is used to generate a detectable signal. For instance, one can use biotin, iminobiotin or desthiobiotin as a tag and then use an avidin or streptavidin conjugate of horseradish peroxidase (HRP) to bind to the tag, and then use a chromogenic substrate (e.g., tetramethylbenzidine) or a fluorogenic substrate such as Amplex Red or Amplex Gold (Molecular Probes, Inc.) to detect the presence of HRP. Similarly, the tag can be a hapten or antigen (e.g., digoxigenin), and an enzymatically, fluorescently, or radioactively labeled antibody can be used to bind to the tag. Numerous labels are known by those of skill in the art and include, but are not limited to, particles, fluorescent dyes, haptens, enzymes and their chromogenic, fluorogenic, and chemiluminescent substrates, and other labels that are described in the Molecular Probes Handbook Of Fluorescent Probes And Research Chemicals by Richard P. Haugland, 6th Ed., (1996), and its subsequent 7th edition and 8th edition updates issued on CD Rom in November 1999 and May 2001, respectively, and in other published sources.

A fluorophore is any chemical moiety that exhibits an absorption maximum beyond 280 nm, and when covalently attached to a labeling reagent retains its spectral properties. Fluorophores include, without limitation; a pyrene (including any of the corresponding derivative compounds disclosed in U.S. Pat. No. 5,132,432), an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine (including any corresponding compounds in U.S. Ser. Nos. 09/968,401 and 09/969,853), a carbocyanine (including any corresponding compounds in U.S. Ser. Nos. 09/557,275; 09/969,853 and 09/968,401; U.S. Pat. Nos. 4,981,977; 5,268,486; 5,569,587; 5,569,766; 5,486,616; 5,627,027; 5,808,044; 5,877,310; 6,002,003; 6,004,536; 6,008,373; 6,043,025; 6,127,134; 6,130,094; 6,133,445; and publications WO 02/26891, WO 97/40104, WO 99/51702, WO 01/21624; EP 1 065 250 A1), a carbostyryl, a porphyrin, a salicylate, an anthranilate, an azulene, a perylene, a pyridine, a quinoline, a borapolyazaindacene (including any corresponding compounds disclosed in U.S. Pat. Nos. 4,774,339; 5,187,288; 5,248,782; 5,274,113; and 5,433,896), a xanthene (including any corresponding compounds disclosed in U.S. Pat. No. 6,162,931; 6,130,101; 6,229,055; 6,339,392; 5,451,343 and U.S. Ser. No. 09/922,333), an oxazine (including any corresponding compounds disclosed in U.S. Pat. No. 4,714,763) or a benzoxazine, a carbazine (including any corresponding compounds disclosed in U.S. Pat. No. 4,810,636), a phenalenone, a coumarin (including an corresponding compounds disclosed in U.S. Pat. Nos. 5,696,157; 5,459,276; 5,501,980 and 5,830,912), a benzofuran (including an corresponding compounds disclosed in U.S. Pat. Nos. 4,603,209 and 4,849,362) and benzphenalenone (including any corresponding compounds disclosed in U.S. Pat. No. 4,812,409) and derivatives thereof. As used herein, oxazines include resorufins (including any corresponding compounds disclosed in U.S. Pat. No. 5,242,805), aminooxazinones, diaminooxazines, and their benzo-substituted analogs.

When the fluorophore is a xanthene, the fluorophore is optionally a fluorescein, a rhodol (including any corresponding compounds disclosed in U.S. Pat. Nos. 5,227,487 and 5,442,045), or a rhodamine (including any corresponding compounds in U.S. Pat. Nos. 5,798,276; 5,846,737; U.S. Ser. No. 09/129,015). As used herein, fluorescein includes benzo- or dibenzofluoresceins, seminaphthofluoresceins, or naphthofluoresceins. Similarly, as used herein rhodol includes seminaphthorhodafluors (including any corresponding compounds disclosed in U.S. Pat. No. 4,945,171). Alternatively, the fluorophore is a xanthene that is bound via a linkage that is a single covalent bond at the 9-position of the xanthene. Preferred xanthenes include derivatives of 3H-xanthen-6-ol-3-one attached at the 9-position, derivatives of 6-amino-3H-xanthen-3-one attached at the 9-position, or derivatives of 6-amino-3H-xanthen-3-imine attached at the 9-position. Preferred fluorophores include xanthene (rhodol, rhodamine, fluorescein and derivatives thereof) coumarin, cyanine, pyrene, oxazine and borapolyazaindacene. Most preferred are sulfonated xanthenes, fluorinated xanthenes, sulfonated coumarins, fluorinated coumarins and sulfonated cyanines. The choice of the fluorophore attached to the labeling reagent will determine the absorption and fluorescence emission properties of the labeling reagent and immuno-labeled complex. Physical properties of a fluorophore label include spectral characteristics (absorption, emission and stokes shift), fluorescence intensity, lifetime, polarization and photo-bleaching rate all of which can be used to distinguish one fluorophore from another.

Typically the fluorophore contains one or more aromatic or heteroaromatic rings, that are optionally substituted one or more times by a variety of substituents, including without limitation, halogen, nitro, cyano, alkyl, perfluoroalkyl, alkoxy, alkenyl, alkynyl, cycloalkyl, arylalkyl, acyl, aryl or heteroaryl ring system, benzo, or other substituents typically present on fluorophores known in the art.

The fluorophore can have an absorption maximum beyond 480 nm. Preferably, the fluorophore absorbs at or near 488 nm to 514 nm (particularly suitable for excitation by the output of the argon-ion laser excitation source) or near 546 nm (particularly suitable for excitation by a mercury arc lamp).

Preferably the detection moiety is a fluorescent dye. The fluorescent dye include for example Fluorescein, Rhodamine, Texas Red, Cy2, Cy3, Cy5, Cy0, Cy0.5, Cy1, Cy1.5, Cy3.5, Cy7, VECTOR Red, ELF^{™} (Enzyme-Labeled Fluorescence), FluorX, Calcein, Calcein-AM, CRYPTOFLUOR^{™}'S, Orange (42 kDa), Tangerine (35 kDa), Gold (31 kDa), Red (42 kDa), Crimson (40 kDa), BHMP, BHDMAP, Br-Oregon, Lucifer Yellow, Alexa dye family, N-(6-(7-nitrobenz-2-oxa-1,3-diazol-4-yl)amino)caproyl) (NBD), BODIPY^{™}, boron dipyrromethene difluoride, Oregon Green, MITOTRACKER^{™} Red, DiOC₇ (3), DiIC₁₈, Phycoerythrin, Phycobiliproteins BPE (240 kDa) RPE (240 kDa) CPC (264 kDa) APC (104 kDa), Spectrum Blue, Spectrum Aqua, Spectrum Green, Spectrum Gold, Spectrum Orange, Spectrum Red, NADH, NADPH, FAD, Infra-Red (IR) Dyes, Cyclic GDP-Ribose (cGDPR), Calcofluor White, Tyrosine and Tryptophan.

Many of fluorophores can also function as chromophores and thus the described fluorophores are also preferred chromophores.

In addition to fluorophores, enzymes also find use as detectable moieties. Enzymes are desirable detectable moieties because amplification of the detectable signal can be obtained resulting in increased assay sensitivity. The enzyme itself does not produce a detectable response but functions to break down a substrate when it is contacted by an appropriate substrate such that the converted substrate produces a fluorescent, colorimetric or luminescent signal. Enzymes amplify the detectable signal because one enzyme on a labeling reagent can result in multiple substrates being converted to a detectable signal. This is advantageous where there is a low quantity of target present in the sample or a fluorophore does not exist that will give comparable or stronger signal than the enzyme. However, fluorophores are most preferred because they do not require additional assay steps and thus reduce the overall time required to complete an assay. The enzyme substrate is selected to yield the preferred measurable product, e.g. colorimetric, fluorescent or chemiluminescence. Such substrates are extensively used in the art, many of which are described in the MOLECULAR PROBES HANDBOOK, supra.

Colorimetric or fluorogenic substrate and enzyme combination uses oxidoreductases such as horseradish peroxidase and a substrate such as 3,3'-diaminobenzidine (DAB) and 3-amino-9-ethylcarbazol-e (AEC), which yield a distinguishing color (brown and red, respectively). Other colorimetric oxidoreductase substrates that yield detectable products include, but are not limited to: 2,2-azino-bis(3-ethylbenzothiaz-oline-6-sulfonic acid) (ABTS), o-phenylenediamine (OPD), 3,3',5,5'-tetramethylbenzidine (TMB), o-dianisidine, 5-aminosalicylic acid, 4-chloro-1-naphthol. Fluorogenic substrates include, but are not limited to, homovanillic acid or 4-hydroxy-3-methoxyphenylacetic acid, reduced phenoxazines and reduced benzothiazines, including Amplexe Red reagent and its variants (U.S. Pat. No. 4,384,042) and reduced dihydroxanthenes, including dihydrofluoresceins (U.S. Pat. No. 6,162,931) and dihydrorhodamines including dihydrorhodamine 123. Peroxidase substrates that are tyramides (U.S. Pat. Nos. 5,196,306; 5,583,001 and 5,731,158) represent a unique class of peroxidase substrates in that they can be intrinsically detectable before action of the enzyme but are "fixed in place" by the action of a peroxidase in the process described as tyramide signal amplification (TSA). These substrates are extensively utilized to label targets in samples that are cells, tissues or arrays for their subsequent detection by microscopy, flow cytometry, optical scanning and fluorometry.

Additional colorimetric (and in some cases fluorogenic) substrate and enzyme combination use a phosphatase enzyme such as an acid phosphatase, an alkaline phosphatase or a recombinant version of such a phosphatase in combination with a colorimetric substrate such as 5-bromo-6-chloro-3-indolyl phosphate (BCIP), 6-chloro-3-indolyl phosphate, 5-bromo-6-chloro-3-indolyl phosphate, p-nitrophenyl phosphate, or o-nitrophenyl phosphate or with a fluorogenic substrate such as 4-methylumbelliferyl phosphate, 6,8-difluoro-7-hydroxy4-methylcoumarinyl phosphate (DiFMUP, U.S. Pat. No. 5,830,912) fluorescein diphosphate, 3-0-methylfluorescein phosphate, resorufin phosphate, 9H-(1,3-dichloro-9,9-dimethylacridin-2-one-7-yl) phosphate (DDAO phosphate), or ELF 97, ELF 39 or related phosphates (U.S. Pat. Nos. 5,316,906 and 5,443,986).

Glycosidases, in particular beta-galactosidase, beta-glucuronidase and beta-glucosidase, are additional suitable enzymes. Appropriate colorimetric substrates include, but are not limited to, 5-bromo4-chloro-3-indolyl beta-D-galactopyranoside (X-gal) and similar indolyl galactosides, glucosides, and glucuronides, o-nitrophenyl beta-D-galactopyranoside (ONPG) and p-nitrophenyl beta-D-galactopyranosid-e. Preferred fluorogenic substrates include resorufin beta-D-galactopyranoside, fluorescein digalactoside (FDG), fluorescein diglucuronide and their structural variants (U.S. Pat. Nos. 5,208,148; 5,242,805; 5,362,628; 5,576,424 and 5,773,236), 4-methylumbelliferyl beta-D-galactopyranoside, carboxyumbelliferyl beta-D-galactopyranoside and fluorinated coumarin beta-D-galactopyranosides (U.S. Pat. No. 5,830,912).

Additional enzymes include, but are not limited to, hydrolases such as cholinesterases and peptidases, oxidases such as glucose oxidase and cytochrome oxidases, and reductases for which suitable substrates are known.

Enzymes and their appropriate substrates that produce chemiluminescence are preferred for some assays. These include, but are not limited to, natural and recombinant forms of luciferases and aequorins. Chemiluminescence-producing substrates for phosphatases, glycosidases and oxidases such as those containing stable dioxetanes, luminol, isoluminol and acridinium esters are additionally useful. For example, the enzyme is luciferase or aequorin. The substrates are luciferine, ATP, Ca⁺⁺ and coelenterazine.

In addition to enzymes, haptens such as biotin are useful detectable moieties. Biotin is useful because it can function in an enzyme system to further amplify the detectable signal, and it can function as a tag to be used in affinity chromatography for isolation purposes. For detection purposes, an enzyme conjugate that has affinity for biotin is used, such as avidin-HRP. Subsequently a peroxidase substrate is added to produce a detectable signal. Haptens also include hormones, naturally occurring and synthetic drugs, pollutants, allergens, affector molecules, growth factors, chemokines, cytokines, lymphokines, amino acids, peptides, chemical intermediates, or nucleotides.

A detectable moiety is a fluorescent protein. Exemplary fluorescent proteins include green fluorescent protein (GFP) the phycobiliproteins and the derivatives thereof, luciferase or aequorin. The fluorescent proteins, especially phycobiliprotein, are particularly useful for creating tandem dye labeled labeling reagents. These tandem dyes comprise a fluorescent protein and a fluorophore for the purposes of obtaining a larger stokes shift wherein the emission spectra is farther shifted from the wavelength of the fluorescent protein's absorption spectra. This is particularly advantageous for detecting a low quantity of a target in a sample wherein the emitted fluorescent light is maximally optimized, in other words little to none of the emitted light is reabsorbed by the fluorescent protein. For this to work, the fluorescent protein and fluorophore function as an energy transfer pair wherein the fluorescent protein emits at the wavelength that the fluorophore absorbs at and the fluorphore then emits at a wavelength farther from the fluorescent proteins than could have been obtained with only the fluorescent protein. A particularly useful combination is the phycobiliproteins disclosed in U.S. Pat. Nos. 4,520,110; 4,859,582; 5,055,556 and the sulforhodamine fluorophores disclosed in U.S. Pat. No. 5,798,276, or the sulfonated cyanine fluorophores disclosed in U.S. Ser. Nos. 09/968/401 and 09/969/853; or the sulfonated xanthene derivatives disclosed in U.S. Pat. No. 6,130,101 and those combinations disclosed in U.S. Pat. No. 4,542,104. Alternatively, the fluorophore functions as the energy donor and the fluorescent protein is the energy acceptor.

Preparation of labeling reagent using low molecular weight reactive dyes is known by those of skill in the art and is well documented, e.g., by Richard P. Haugland, Molecular Probes Handbook Of Fluorescent Probes And Research Chemicals, Chapters 1-3 (1996) and by Brinkley, Bioconjugate Chem. 3, 2 (1992). Labeling proteins typically result from mixing appropriate reactive dyes and the protein to be conjugated in a suitable solvent in which both are soluble. The majority of the preferred dyes are readily soluble in aqueous solutions, facilitating conjugation reactions with most biological materials. For those reactive dyes that are photoactivated, conjugation requires illumination of the reaction mixture to activate the reactive dye.

As used herein, the term "binder" refers to a molecule that may bind to one or more targets in the biological sample. A binder may specifically bind to a target. Suitable binders may include one or more of natural or modified peptides, proteins (e.g., antibodies, affibodies, or aptamers), nucleic acids (e.g., polynucleotides, DNA, RNA, or aptamers); polysaccharides (e.g., lectins, sugars), lipids, enzymes, enzyme substrates or inhibitors, ligands, receptors, antigens, or haptens. A suitable binder may be selected depending on the sample to be analyzed and the targets available for detection. For example, a target in the sample may include a ligand and the binder may include a receptor or a target may include a receptor and the binder may include a ligand. Similarly, a target may include an antigen and the binder may include an antibody or antibody fragment or vice versa. A target may include a nucleic acid and the binder may include a complementary nucleic acid. Both the target and the binder may include proteins capable of binding to each other.

A biological sample may be of prokaryotic origin, archaeal origin, or eukaryotic origin (e.g., insects, protozoa, birds, fish, and reptiles). The biological sample is mammalian (e.g., rat, mouse, cow, dog, donkey, guinea pig, or rabbit). The biological sample is of primate origin (e.g., example, chimpanzee, or human).

As used herein, the term "control probe" refers to an agent having a binder coupled to a signal generator or a signal generator capable of staining directly, such that the signal generator retains at least 80 percent signal after contact with an electron transfer reagent and subsequent irradiation. A suitable signal generator in a control probe is not substantially inactivated, e.g., substantially bleached by photo activated chemical bleaching, when contacted with the electron transfer reagent and irradiated. Suitable examples of signal generators may include a fluorophore that does not undergo bleaching under the conditions employed (e.g. DAPI).

As used herein, the term "enzyme" refers to a protein molecule that can catalyze a chemical reaction of a substrate. A suitable enzyme catalyzes a chemical reaction of the substrate to form a reaction product that can bind to a receptor (e.g., phenolic groups) present in the sample. A receptor may be exogeneous (that is, a receptor extrinsically adhered to the sample or the solid- support) or endogeneous (receptors present intrinsically in the sample or the solid-support). Examples of suitable enzymes include peroxidases, oxidases, phosphatases, esterases, and glycosidases. Specific examples of suitable enzymes include horseradish peroxidase, alkaline phosphatase, β-D-galactosidase, lipase, and glucose oxidase.

As used herein, the term "enzyme substrate" refers to a chemical compound that is chemically catalyzed by an enzyme to form a reaction product. The reaction product is capable of binding to a receptor present in the sample. Enzyme substrates employed in the methods herein may include non-chromogenic or non- chemiluminescent substrates. A signal generator may be attached to the enzyme substrate as a label.

As used herein, the term "electron transfer reagent" refers to a reagent that can engage in a photoreaction with a molecule capable of undergoing photoexcitation. This term also refers to a composition comprising a reagent that can engage in a photoreaction with a molecule capable of undergoing photoexcitation. The molecule capable of undergoing photoexcitation may be a signal generator. The electron transfer reagent may donate an electron to the signal generator in the course of a photoreaction. The electron transfer reagent may accept an electron from the signal generator in the course of a photoreaction.

The electron transfer reagent donating an electron to the signal generator in the course of a photoreaction may be a borate salt including the photo- induced chemical bleaching agent used in the disclosure for quenching eosin fluorescence. The electron transfer reagent accepting an electron from the photoexcited molecule may be an onium salt [e.g., diphenyliodonium hexafluorophosphate (DPI) or dimethylphenacylsulf onium tetrafluoroborate (DMPS)], or tetrabutylammonium butyltriphenylborate (TBAB). An electron transfer reagent may include one or more chemicals that can engage in a photoreaction with a molecule capable of undergoing photoexcitation. The molecule capable of undergoing photoexcitation may be a signal generator. An electron transfer reagent may be contacted with the sample in the form of a solid, a solution, a gel, or a suspension. Other suitable electron transfer reagents may include sulfinates, enolates, carboxylates (e.g., ascorbic acid), organometallics and amines (e.g., triethanolamine, and N-phenylglycine). These and other electron transfer reagents have been previously described (see, e.g., Macromolecules 1974, 7, 179-187; Photogr. Sci. Eng. 1979, 23, 150-154; Topics in Current Chemistry, Mattay, J., Ed.; Springer- Verlag: Berlin, 1990, Vol. 156, pp 199-225; and Pure Appl. Chem. 1984, 56, 1191-1202.). [0060] As used herein, the term "fluorophore" or "fluorescent signal generator" refers to a chemical compound, which when excited by exposure to a particular wavelength of light, emits light at a different wavelength. Fluorophores may be described in terms of their emission profile, or "color." Green fluorophores (for example Cy3, FITC, and Oregon Green) may be characterized by their emission at wavelengths generally in the range of 515-540 nanometers. Red fluorophores (for example Texas Red, Cy5, and tetramethylrhodamine) may be characterized by their emission at wavelengths generally in the range of 590-690 nanometers. Examples of fluorophores include, but are not limited to, 4-acetamido-4'-isothiocyanatostilbene- 2,2'disulfonic acid, acridine, derivatives of acridine and acridine isothiocyanate, 5-(2'-aminoethyl)aminonaphthalene-l-sulfonic acid (EDANS), 4-amino-N-[3-vinylsulfonyl)phenyl]naphthalimide-3,5 disulfonate (Lucifer Yellow VS), N-(4-anilino-l-naphthyl)maleimide, anthranilamide, Brilliant Yellow, coumarin, coumarin derivatives, 7-amino- 4-methylcoumarin (AMC, Coumarin 120), 7-amino-trifluoromethylcouluarin (Coumaran 151), cyanosine; 4',6-diaminidino-2-phenylindole (DAPI), 5',5"-dibromopyrogallol-sulfonephthalein (Bromopyrogallol Red), 7-diethylamino-3-(4'-isothiocyanatophenyl)-4-methylcoumarin, -, 4,4'- diisothiocyanatodihydro-stilbene-2,2'-disulfonic acid, 4,4'-diisothiocyanatostilbene-2,2'- disulfonic acid, 5 -[dimethylamino] naphthalene- 1-sulfonyl chloride (DNS, dansyl chloride), fluorescein and derivatives such as 5-carboxyfluorescein (FAM), 5-(4,6-dichlorotriazin-2-yl) aminofluorescein (DTAF), 2'7'-dimethoxy-4'5'-dichloro-6-carboxyfluorescein (JOE), fluorescein, fluorescein isothiocyanate (FITC), QFITC (XRITC); fluorescamine derivative (fluorescent upon reaction with amines); IR144; IR1446; Malachite Green isothiocyanate; 4-methylumbelliferone; ortho cresolphthalein; nitro tyro sine; pararosaniline; Phenol Red, B-phycoerythrin; o-phthaldialdehyde derivative (fluorescent upon reaction with amines); pyrene and derivatives such as pyrene, pyrene butyrate and succinimidyl 1 -pyrene butyrate; Reactive Red 4 (Cibacron.RTM. Brilliant Red 3B-A), rhodamine and derivatives such as 6-carboxy-X-rhodamine (ROX), 6- carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101 and sulfonyl chloride derivative of sulforhodamine 101 (Texas Red); N,N,N',N'-tetramethyl- 6-carboxyrhodamine (TAMRA); tetramethyl Rhodamine, tetramethyl rhodamine isothiocyanate (TRrrC); riboflavin; rosolic acid and lathanide chelate derivatives, cyanines, pyrelium dyes, squaraines, 1,3-dichloro-7-hydroxy-9,9-dimethyl-2(9H)-Acridinone (DDAO), and dimethylacridinone (DAO). The fluorophore can be cyanine, rhodamine, BODIPY or 1,3-dichloro-7-hydroxy-9,9-dimethyl-2(9H)-Acridinone (DDAO) dyes. The fluorophore is preferably a cyanine dye. The cyanine dye is preferably Cy3 or Cy5.

As used herein the term "H&E stain" generally refers to hematoxylin and eosin Y stain (H&E stain or HE stain). A histological section stained with H&E is often termed "H&E section", "H+E section", or "HE section". The staining method involves application of hemalum, which is a complex formed from aluminum ions and oxidized haematoxylin. These colors nuclei of cells (and a few other objects, such as keratohyalin granules) blue. The nuclear staining is followed by counterstaining with an aqueous or alcoholic solution of eosin Y, which colors other, eosinophilic structures in various shades of red, pink and orange.

The staining of nuclei by hemalum does not require the presence of DNA and is probably due to binding of the dye-metal complex to arginine-rich basic nucleoproteins such as histones. The eosinophilic structures are generally composed of intracellular or extracellular protein. The Lewy bodies and Mallory bodies are examples of eosinophilic structures. Most of the cytoplasm is eosinophilic. Red blood cells are stained intensely red. Thus, the term "H&E stain" also encompasses the use of these eosin Y analogues in obtaining a histological stain. These alternatives to eosin Y have lower intrinsic fluorescence and may be preferred for certain staining procedures.

As used herein the term charge transfer reagent refers to a chemical reagent that can form a charge transfer complex with eosin and in the process quenches eosin fluorescence. Examples of charge transfer reagents include but are not limited to p-quat, di-quat, phenylene diamine dihydrochloride.

As used herein, the term "in situ" generally refers to an event occurring in the original location, for example, in intact organ or tissue or in a representative segment of an organ or tissue. In situ analysis of targets may be performed on cells derived from a variety of sources, including an organism, an organ, tissue sample, or a cell culture. In situ analysis provides contextual information that may be lost when the target is removed from its site of origin. Accordingly, in situ analysis of targets describes analysis of target-bound probe located within a whole cell or a tissue sample, whether the cell membrane is fully intact or partially intact where target-bound probe remains within the cell. Furthermore, the methods disclosed herein may be employed to analyze targets in situ in cell or tissue samples that are fixed or unfixed.

As used herein, the terms "irradiation" or "irradiate" refer to act or process of exposing a sample or a solution to non-ionizing radiation. The nonionizing irradiation may have wavelengths between 350 nm and 1.3 µιη. The non-ionizing radiation may be visible light of 400-700 nm in wavelength. Irradiation may be accomplished by exposing a sample or a solution to a radiation source, e.g., a lamp, capable of emitting radiation of a certain wavelength or a range of wavelengths. A molecule capable of undergoing photoexcitation is photoexcited as a result of irradiation. The molecule capable of undergoing photoexcitation is a signal generator, e.g., a fluorescent signal generator. Irradiation of a fluorescent signal generator initiates a photoreaction between the fluorescent signal generator and the electron transfer reagent. Irradiation initiates a photoreaction substantially inactivates the signal generator by photoactivated chemical bleaching.

Optical filters may be used to restrict irradiation of a sample or a solution to a particular wavelength or a range of wavelengths. The optical filters may be used to restrict irradiation to a narrow range of wavelengths for selective photoexcitation of one or more molecules capable of undergoing photoexcitation. The term "selective photoexcitation" refers to an act or a process, whereby one or more molecules capable of undergoing photoexcitation are photoexcited in the presence of one or more other molecules capable of undergoing photoexcitation that remain in the ground electronic state after irradiation.

The molecule capable of undergoing photoexcitation is a fluorescent dye, e.g., a cyanine dye. Irradiation limited to a range of wavelengths between 520-580 nm is used for selective photoexcitation of a Cy3 dye. Irradiation limited to a range of wavelengths between 620-680 nm is used for selective photoexcitation of a Cy5 dye. Irradiation of a sample at a specific wavelength may also be accomplished by using a laser.

As used herein, the term "peroxidase" refers to an enzyme class that catalyzes an oxidation reaction of an enzyme substrate along with an electron donor. Examples of peroxidase enzymes include horseradish peroxidase, cytochrome C peroxidase, glutathione peroxidase, microperoxidase, myeloperoxidase, lactoperoxidase, or soybean peroxidase.

As used herein, the term "peroxidase substrate" refers to a chemical compound that is chemically catalyzed by peroxidase to form a reaction product. Peroxidase substrates may include non-chromogenic or non- chemiluminescent substrates. A fluorescent signal generator may be attached to the peroxidase substrate as a label.

As used herein, the term "bleaching", "photo activated chemical bleaching" or "photoinduced chemical bleaching" refers to an act or a process whereby a signal generated by a signal generator is modified in the course of a photoreaction. The signal generator is irreversibly modified.

The signal may be diminished or eliminated as a result of photoactivated chemical bleaching. The signal generator may be completely bleached, i.e., the signal intensity decreases by about 100%. The signal may be an optical signal, and the signal generator may be an optical signal generator. The term "photoactivated chemical bleaching" is meant to exclude photobleaching, or loss of signal (e.g., fluorescent signal) that may occur in the absence of electron transfer reagent, e.g., after continued irradiation of a signal generator, such as a fluorophore, or after its continued exposure to light. As used herein, the term "photoexcitation" refers to an act or a process whereby a molecule transitions from a ground electronic state to an excited electronic state upon absorption of radiation energy, e.g. upon irradiation. Photoexcited molecules can participate in chemical reactions, e.g., in electron transfer reactions. A molecule capable of undergoing photoexcitation is a signal generator, e.g., a fluorescent signal generator.

As used herein, the term "photoreaction" or a "photoinduced reaction" refers to a chemical reaction that is initiated and/or proceeds as a result of photoexcitation of at least one reactant. The reactants in a photoreaction may be an electron transfer reagent and a molecule capable of undergoing photoexcitation. A photoreaction may involve an electron transfer from the electron transfer reagent to the molecule that has undergone photoexcitation, i.e., the photoexcited molecule. A photoreaction may also involve an electron transfer from the molecule that has undergone photoexcitation to the electron transfer reagent. The molecule capable of undergoing photoexcitation is a fluorescent signal generator, e.g., a fluorophore. Photoreaction results in irreversible modification of one or more components of the photoreaction. Photoreaction substantially inactivates the signal generator by photoactivated chemical bleaching.

The photoreaction may involve intermolecular electron transfer between the electron transfer reagent and the photoexcited molecule, e.g., the electron transfer occurs when the linkage between the electron transfer reagent and the photoexcited molecule is transitory, forming just prior to the electron transfer and disconnecting after electron transfer.

The photoreaction may involve intramolecular electron transfer between the electron transfer reagent and the photoexcited molecule, e.g. the electron transfer occurs when the electron transfer reagent and the photoexcited molecule have been linked together, e.g., by covalent or electrostatic interactions, prior to initiation of the electron transfer process. The photoreaction involving the intramolecular electron transfer can occur, e.g., when the molecule capable of undergoing photoexcitation and the electron transfer reagent carry opposite charges and form a complex held by electrostatic interactions. For example, a cationic dye, e.g., a cationic cyanine dye and triphenylbutyl borate anion may form a complex, wherein intramolecular electron transfer may occur between the cyanine and borate moieties upon irradiation.

As used herein, the term "probe" refers to an agent having a binder and a label, such as a signal generator or an enzyme. In some embodiments, the binder and the label (signal generator or the enzyme) are embodied in a single entity. The binder and the label may be attached directly (e.g., via a fluorescent molecule incorporated into the binder) or indirectly (e.g., through a linker) and applied to the biological sample in a single step. In alternative embodiments, the binder and the label are embodied in discrete entities (e.g., a primary antibody capable of binding a target and an enzyme or a signal generator-labeled secondary antibody capable of binding the primary antibody). When the binder and the label (signal generator or the enzyme) are separate entities they may be applied to a biological sample in a single step or multiple steps. As used herein, the term "fluorescent probe" refers to an agent having a binder coupled to a fluorescent signal generator. The probe may comprise an optical signal generator, such that the signal observed/detected is an optical signal. The probe may comprise a fluorescent signal generator, such that the signal observed/detected is a fluorescent signal.

As used herein, the term "signal generator" refers to a molecule capable of providing a detectable signal using one or more detection techniques (e.g., spectrometry, calorimetry, spectroscopy, or visual inspection). Suitable examples of a detectable signal may include an optical signal, and electrical signal. Examples of signal generators include one or more of a chromophore, a fluorophore, or a Raman-active tag. As stated above, with regard to the probe, the signal generator and the binder may be present in a single entity (e.g., a target binding protein with a fluorescent label). Alternatively, the binder and the signal generator may be discrete entities (e.g., a receptor protein and a labeled-antibody against that particular receptor protein) that associate with each other before or upon introduction to the sample.

The signal generator may be an optical signal generator. The optical signal generator may be a fluorescent signal generator, e.g., a fluorophore. The fluorescent signal generator may be a cyanine dye, e.g., Cy3, Cy5 or Cy7. The signal generator, e.g., a fluorophore, may be charged. The signal generator may be a cationic fluorescent dye.

As used herein, the term "solid support" refers to an article on which targets present in the biological sample may be immobilized and subsequently detected by the methods disclosed herein. Targets may be immobilized on the solid support by physical adsorption, by covalent bond formation, or by combinations thereof. A solid support may include a polymeric, a glass, or a metallic material. Examples of solid supports include a membrane, a microtiter plate, a bead, a filter, a test strip, a slide, a cover slip, and a test tube.

As used herein, the term "specific binding" refers to the specific recognition of one of two different molecules for the other compared to substantially less recognition of other molecules. The molecules may have areas on their surfaces or in cavities giving rise to specific recognition between the two molecules arising from one or more of electrostatic interactions, hydrogen bonding, or hydrophobic interactions. Specific binding examples include, but are not limited to, antibody-antigen interactions, enzyme- substrate interactions, polynucleotide interactions, and the like. A binder molecule may have an intrinsic equilibrium association constant (KA) for the target no lower than about 105 M-1 under ambient conditions such as a pH of about 6 to about 8 and temperature ranging from about 0 °C to about 37 °C.

As used herein, the term "target" refers to the component of a biological sample that may be detected when present in the biological sample. The target may be any substance for which there exists a naturally occurring specific binder (e.g., an antibody), or for which a specific binder may be prepared (e.g., a small molecule binder or an aptamer). In general, a binder may bind to a target through one or more discrete chemical moieties of the target or a three-dimensional structural component of the target (e.g., 3D structures resulting from peptide folding). The target may include one or more of natural or modified peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. Targets may include proteins or nucleic acids.

### KITS (not part of the invention)

The present disclosure also provides kits comprising the components used in the method according to the invention. The kit includes one or more components including, but not limited to stains such as 3-amino-9-ethylcarbazole: (AEC), and/or 3,3'-Diaminobenzidine: (DAB), suitable buffers, and destains such as ethanol or xylene, as discussed herein, in association with one or more additional components including, but not limited to a carrier and/or an immunotherapy agent or chemotherapeutic agent, as discussed herein. In certain embodiments, MICSSS is combined with in situ hybridization (FISH or CISH) using DNA or RNA probes. Thus, kit components useful for performing *in situ* hybridization (FISH or CISH) using DNA or RNA probes may be combined with MICSSS components.

A kit includes a stain in one container (*e.g.,* in a sterile glass or plastic vial) and a destaining agent in another container (*e.g.,* in a sterile glass or plastic vial). Additional components include buffers and destaining agents such as ethanol or xylene.

If the kit includes a pharmaceutical composition, an immunotherapy agent or chemotherapeutic agent for parenteral administration to a subject, the kit can include a device for performing such administration. For example, the kit can include one or more hypodermic needles or other injection devices as discussed above.

The kit can include a package insert including information concerning the label compositions and sequential staining methods in the kit. For example, any one or combination of the following information may be supplied in the insert: automated imaging and image processing, and image storage, pharmacokinetics, pharmacodynamics, clinical studies, efficacy parameters, indications and usage, contraindications, warnings, precautions, adverse reactions, overdosage, proper dosage and administration, how supplied, proper storage conditions, references, manufacturer/distributor information and patent information.

### GENERAL METHODS

Standard methods in molecular biology are described in Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described (see, *e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protocols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra*)*.* Standard techniques for characterizing ligand/receptor interactions are available (see, *e.g.,* Coligan, et al. (2001) Current Protocols in Immunology, Vol. 4, John Wiley, Inc., New York).

Several novel cutting edge methods for high dimensional tissue analysis have been developed, but all have significant practical limitations to broad implementation in pathology labs (19,22). Tissue mass cytometry which could optimally stain for large Ab panels requires tissue vaporization, forbidding slide storage, have low resolution (1 micrometer), low sample throughput due to slow image acquisition and will remain for some time at least restricted to selected academic centers. Other multiplex platforms such as Vectra (Perkin Elmer) or MultiOmyx (GE Healthcare) have inherent limitations that include the use of costly material and fluorescent dyes that are light sensitive and induce spectral overlap, low sample throughput due to slow whole-slide image scanning, and image analyses limited to defined fields.

The need for monitoring of tissue inflammatory lesions in response to the flurry of novel immunomodulation strategies for the treatment of cancer and inflammatory disease has never been more pressing (17, 24). In cancer in particular, immunotherapy strategies have lead to significant clinical responses, yet these responses remain limited to a subset of patients and the mechanisms that lead to response or no response to immunotherapy agents remain elusive (25, 26). Recent results have revealed that in patients responding to checkpoint blockade, T cells infiltrate the center of the tumors, whereas T cells remain at the edge, often associated with macrophages and dense network of fibroblasts, in patients that do not respond to checkpoint blockade (15, 16). These results highlight the need for the inclusion of longitudinal high dimensional analysis of tissue lesions in immune monitoring strategies and MICSSS is positioned to meet this need.

### EXAMPLES

### Development of a novel multiplexed IHC method on single paraffin-embedded tissue slides

Since a very limited number of chromogens can be used concomitantly on one same tissue slide, due to the paucity of available enzymatic substrates, the present tests were carried out to determine whether consecutive cycles of Ab staining, image scanning, destaining of chromogen, blocking, and restaining would function to characterize the complexity of the TME **(****Figure 1A****).** Thus, one single slide of FFPE tumor tissue section was first stained with a standard primary Ab followed by a biotin-linked secondary Ab and horseradish peroxidaseconjugated streptavidin to amplify the signal. Peroxidase-labeled compounds were revealed using 3-amino-9-ethylcarbazole (AEC), an aqueous substrate that results in red staining, and counterstained using hematoxylin (blue). The slide was mounted for microscopy and scanned at high resolution by digital imaging. The colored reaction product was then removed using an organic solvent-based destaining buffer after coverslip removal. **Figure 1B** shows a CD20 B cell follicle staining followed by a chemical destaining. Because the Abs were not stripped completely by the destaining or the antigen retrieval steps, as shown by the ability to restain slides directly with AEC substrate, the destained slide was treated with a protein blocking buffer before the next cycle of staining, to prevent any remnant reactivity to primary or secondary antibodies used in the first cycle. This allowed performing up to ten cycles of staining/image scanning/destaining on the same FFPE tissue slide, as shown in Figure 1B. The destaining/staining/scanning process took 6-7 h per cycle and at least 30 samples were concomitantly processed manually, whereas a much higher number of slides could be processed with staining automation.

### MICSSS helps characterize the spatial distribution of complex cell populations in tumor tissues without cross-reactivity between iterative staining cycles.

To examine whether the MICSSS assay can be used to comprehensively assess the diversity of the immune microenvironment of tumor tissues, FFPE colorectal tumor tissue lesions were stained with hematoxylin together with markers of diverse immune cell lineages, including markers of lymphoid and myeloid cells. The T cell markers CD2, CD3 and CD8, the B cell marker CD20 were used, as well as the transcription factor enriched in T regulatory cells Foxp3 (forkhead box P3), the myeloid cell marker DC-LAMP, and the nuclear proliferation marker Ki-67 to assess the functional state of T and B cell populations. Antibodies were applied consecutively and the slides were scanned after each staining following the method described in Figure 1A. For each marker, a virtual layer was selected based on chromogen color from individual captured images. Each layer was assigned an artificial color to help distinguish different markers when overlaid in a composite figure. In order to improve the visualization of colocalized markers, bright field images were inverted in image processing software and red green blue (RGB) channels separated to generate fluorescent-like images.

As shown in **Figure 2****,** the expression of nuclear, cytoplasmic and membranous markers can be assessed independently or simultaneously to identify complex cell populations such as CD3+ CD8- FoxP3+ Ki-67+ T cells. Using the Manders' overlap coefficient (tM) with threshold set by Costes method as a measure of colocalization, a high degree of colocalization was observed between markers known to be expressed by the same cellular compartment (e.g. tM(CD8/CD3)=0.864; tM(CD3/CD2)=0.887; tM(CD8/CD2)=0.842), and a low degree of colocalization between markers expressed by different immune cell populations (e.g. tM(CD20/CD3)=0.063; tM(CD20/CD8)=0.066; tM(CD20/DC-LAMP)=0.023).

Importantly, no cross reactivity was observed between secondary Abs targeting primary Abs from the same species or with the same isotype. Absence of cross-reactivity was dependent on incubation with a blocking buffer prior to each re-staining step.

### MICSSS surprisingly does not decrease antigenicity or generate steric hindrance

There is a concern that a repetitive or sequential destaining/restaining method could lead to potential alteration of tissue integrity and antigen expression. To address this question, serial FFPE colorectal cancer tissue sections were stained and shuffled the order of the primary Abs used for iterative staining cycles on each slide. The various markers were quantified on these serial sections, and no significant differences were observed in the density of positive cells found in untreated slides and slides that underwent several staining, destaining, and restaining cycles **(****Figures 3A-B****)** indicating that tissue antigen expression was not affected during the staining and destaining process. Of note however, markers with low and heterogeneous expression level (e.g. PD-L1) could be affected by numerous cycles of staining/destaining and should be prioritized in the staining. Thus, in certain situations for low, sensitive, or heterogeneous markers, there could be an optimum staining/destaining cycle of 2-3 cycles, or fewer than 5 cycles. For stable antigens, the number of staining/destaining cycles can be any number from 2 to at least 10, and in certain instances more than 10 staining/destaining cycles are effective.

To address whether consecutive destaining can alter signal intensity, serial sections were stained again with a shuffled Ab sequence following the MICSSS workflow. After image acquisition, the pictures were subjected to color deconvolution. Then, the intensity histograms of pixels corresponding to chromogen were analyzed, revealing similar signal intensities after several cycles of destaining/restaining cycles **(****Figure 4****),** and establishing that MICSSS does not significantly alter the antigenicity of any of the markers tested. Additionally, these results demonstrate that destained slides can be successfully restained even after several months of storage, allowing prolonged slide storage for future use as new markers become available. Slides have been stored for at least 6 months at 4°C and found to still be amenable to the present MICSSS methods.

Another potential caveat of repetitive Ab incubation is the potential associated steric hindrance due to remaining Abs. In order to address this issue, different antigens expressed by the same cellular compartments were stained using the MICSSS method. Importantly, the staining data in Figs. S3A-C demonstrate the ability to detect multiple markers expressed on the same cell including CD2+ CD3+ CD8+ triple positive T cells, CD2+ CD3+ CD8- PD-1+ T follicular helper cells **(****Figure 5A****)** and HLA-DR+ CD206+ CD68+ triple positive macrophages **(****Figure 5B****).** No steric hindrance was observed as we were able to visualize cytoplasmic, nuclear and cellular markers in the same cellular compartment **(****Figure 5B****).** Absence of steric hindrance was further confirmed upon successful consecutive/sequential cycles of staining/bleaching using the same marker **(****Figure 6****).**

### MICSSS can be generated while preserving a fixed diagnostic marker.

Pathology rules and regulations at some institutions may require storing stained slides for prolonged periods of time, thus preventing the destaining of diagnostic markers. To address whether such diagnostic markers could be preserved while generating multiple consecutive stainings, we developed a destaining procedure that will allow us to remove the AEC stain without affecting other chromogens. **Figure 7** shows lung tumor FFPE tissue section stained with anti-cytokeratins Ab and revealed with the chromogen DAB (an example of a fixed diagnostic marker). In the next step, the slide was stained/destained consecutively for B cells (CD20), marker of cell proliferation (Ki-67), mature dendritic cells (DC-LAMP) and plasma cells (CD138) and revealed with the AEC chromogen. The fixed marker cytokeratin/DAB stain remained untouched while the AEC stain was removed after each staining cycle confirming that the MICSSS method can be used even if long-term storage of diagnostic marker stained-slides is required.

### MICSSS facilitates profiling tumor response to checkpoint blockade

Recent studies of tumor lesions treated with checkpoint blockade antibodies have highlighted the need to assess immune cell distribution and phenotype in the TME, based on their predictive value for clinical benefit (15, 16). In order to determine whether MICSSS could be used effectively to track longitudinal immune cell changes in tumor lesions treated with immunotherapy regimen, pre- and post- treatment tissue was analyzed. Specifically, 5 pre-post treatment tissue pairs obtained from 5 cutaneous melanoma patients prior and after treatment with anti-CTLA-4 monoclonal Ab (ipilimumab) were stained and analyzed using MICSSS.

Tumor tissue sections were stained for PD-L1, myeloid (CD68 and DC-LAMP) and lymphoid populations (CD20, CD3 and FoxP3). Stained sections were scanned and pictures analyzed using image processing software and quantified using CellProfiler **(Table 1).** The results showed that PD-L1 staining was heterogeneous between patients **(****Figure 8A****)** expressed both on tumor cells and tumor-associated CD68+ macrophages and DC-LAMP+ mature DCs **(****Figures 8B and 8C****).** Multi-parameter image analysis revealed a wide range of PD-L1 expression on macrophages (5-90%) and mature DCs (0-90%) **(Table 1).** Ectopic lymphoid structures were observed (also called tertiary-lymphoid structures) in 4 out of 10 tissue samples. These structures were organized in B-cell follicles, adjacent to T-cell areas, and contained antigen-presenting cells including CD68+ macrophages and DC-LAMP+ mature DCs **(****Figure 8A****).** Even with this limited sample number, the data show that MICSSS can be used to track changes in complex immune subsets *in situ* throughout therapy.

**Table 1: Comparative immunohistochemical analysis of melanoma lesions pre and post-treatment with ipilimumab**

| | **Responders (n=3)** | | **Non-Responders (n=2)** | |
|---|---|---|---|---|
| | Pre-ipilimumab | Post-ipilimumab | Pre-ipilimumab | Post-ipilimumab |
| **CD3⁺ (cells/mm²)** | **3542** | **2998** | **1657** | **1785** |
| Mean [min-max] | [44-6375] | [357-5005] | [100-3214] | [26-3544] |
| **CD3⁺Foxp3⁺ (cells/mm²)** | **150** | **256** | **125** | **320** |
| Mean [min-max] | [3-442] | [162-420] | [50-200] | [3-637] |
| **CD20⁺ (cells/mm²)** | **472** | **574** | **73** | **754** |
| Mean [min-max] | [1-1411] | [112-1031] | [32-113] | [2-1514] |
| **CD68⁺ (cells/mm²)** | **574** | **773** | **1138** | **495** |
| Mean [min-max] | [212-1040] | [716-850] | [1085-1190] | [98-891] |
| DC- **LAMP⁺ (cells/mm²)** | **40** | **20** | **18** | **11** |
| Mean [min-max] | [1-115] | [7-28] | [14-21] | [1-20] |
| **CD68⁺PD-L1⁺** (%) | **43** | **53** | **55.5** | **23.5** |
| Mean [min-max] | [5-90] | [30-90] | [38-73] | [8-39] |
| **DC-LAM⁺PD-L1⁺ (%)** | **20** | **76** | **45.5** | **34.5** |
| Mean [min-max] | [0-35] | [66-90] | [44-47] | [0-69] |

### MICSSS can identify novel immune prognostic markers in cancer patients

To determine whether MICSSS can be used for the identification of novel immune prognostic markers in lung cancer, tissue cores obtained from the center of tumors isolated from 75 non-small cell lung cancer (NSCLC) patients were analyzed on a single slide in a tissue microarray (TMA) format. The tissue cores were stained with a 10-plex marker panel that included CD3 (marker of all lymphocytes), CD20 (marker of B cells), FoxP3 (marker of regulatory/activated T cells), CD68 (marker of macrophages), CD66b (marker of neutrophils), DC-LAMP (marker of mature DCs), CDlc (marker of DC and B cell subsets), MHC class I, Ki-67 (marker of cell proliferation) and cytokeratin (marker of normal and neoplastic tissue of epithelial origin).

The MICSSS analyses helped revealing significant inter-individual heterogeneity in the density of tumor infiltrating immune cells, as previously reported (4). Representative examples of tumors with high or low CD3, CD20, FoxP3, CD68, CD66b, DC-LAMP, CDlc and Ki-67 positive cell densities as well as high and low MHC class I expression are shown in **Figure 9A****.** Statistically significant correlations were also found between patients' overall survival and density of tumor-associated CD3+ (p=0.0046), Foxp3+ (p=0.01), CD68+ (p=0.036), CD66b+ (p=0.046), DC-LAMP+ (p<0.0001) and CD1c+ (p=0.008) cells **(****Figure 9B****).** Co-expression analyses showed that CDlc was found on both B cells and DCs but that the prognostic value of CD1c+ cells was mostly attributable to DCs (CD1c+ CD20- cells) **(****Figure 10A-B****).** Loss of MHC class I expression was a significant indicator of poor prognosis (p=0.049). There was no significant correlation between the density of CD20+ B cells and improved overall survival (p=0.42; **Figure 9B****).** Tumor and immune cell proliferation (Ki-67-T and Ki-67-I, respectively, based on marker colocalization) were not significantly associated with overall survival (p=0.23 and p=0.11, respectively). Combined analysis of the presence of DC-LAMP+ mature DCs and CD66b+ neutrophils **(****Figure 9C****)** in tumors revealed that tumor lesions that were poor in DCs and rich in neutrophils (DC-LAMPlow and CD66bhigh; n=6) correlated with reduced overall survival, whereas DC-LAMPhigh/CD66blow tumors (n=35) correlated with increased overall survival (70% overall survival at 8 years; p<0.0001). The density of tumor associated mature DCs helped sub-categorize early stage patients (TNM stages I and II) and late stage patients (TNM stages III and IV) into good and poor prognosis groups **(****Figure 9C****).** Importantly, analysis of mature DC density helped identify patients with high tumor associated CD3+ T cell densities but poor prognosis **(****Figure 9C****).** Using Cox multivariate regression analyses on this small cohort of patients, patient age, TNM stage and CD66b/DC-LAMP score were significantly and independently associated with overall survival (HR = 2.473, 3.113 and 0.476, and P = 6.78 × 10-3, 2.09 × 10-4 and 4.97 × 10-5 respectively; **Table 2).** These data show that MICSSS can help screen and validate comprehensive panels of prognostic factors or help discover new prognostic markers in a samples paring manner.

**Table 2. Multivariate Cox proportional hazards analyses for overall survival according to clinical parameters and immune cell densities in NSCLC**

| | **HR** | **95% Cl** | **P value** |
|---|---|---|---|
| **TNM stage** (I/II/III/IV) | 2.407 | (1.513-3.828) | 2.09 × 10⁻⁴ |
| **Age** (<60y vs. >60y) | 3.113 | (1.368 - 7.082) | 6.78 × 10⁻³ |
| **CD66b/DC-LAMP score** (LoHi/HiHi/LoLo/HiLo) | 0.474 | (0.330 - 0.680) | 4.97 × 10⁻⁵ |

### Digital cartography of tumors for multi-parameter analysis at the cellular level

An important aspect of the MICSSS assays is to provide a detailed analysis of the composition and spatial distribution of the different cell populations in tissue specimens allowing a digital cartography of the tumor tissue and complex multiparametric description of key cell populations. To perform these analyses in the setting of large clinical trials, it is important to generate a high-throughput and robust image analysis approach. To address this need, an automated spatial alignment of digital whole-slide images of the different stains was developed. For highest robustness, positive cell recognition was implemented using convolutional neural networks, a type of `deep learning' algorithm, and connected component and statistical analysis to extract cell counts for single and multi-positive cells. This analysis provides multiple images containing all pixels positive for each biomarker. These images are then integrated into the desired digital landscape map of the tissue lesion containing a multiparametric description of biomarker-stained positive cells. Complete description of this pipeline is provided in the Methods section. As a proof-of-concept, this methodology was applied to identify single positive CD3 and Ki-67 cells as well as double-positive (CD3+ Ki-67+) proliferating T-cells in the lung cancer TMA. Cells marked computationally in green that are double positive for CD3 and Ki-67 in two different tumors identifying low and high T cell proliferation state were scored manually and using automatic quantification to compare quantification methods. Significant correlations (r=0.907; p=3.4 × 10-29 and r=0.901; p=3.7 × 10-28) were found between manual quantification (done by two independent observers) and fully automatic quantifications using this new automated image software validating the accuracy of the fully integrated approach.

### Conclusions

A simple and highly sensitive multiplexed chromogen-based IHC method, named MICSSS, has been developed to comprehensively characterize tissue cell phenotype, state and spatial distribution in inflammatory lesions. Examples provided herein illustrate the MICSSS methods are suitable for mapping the TME, however, all types of tissues can be thoroughly analyzed using the same approach, or slight variations thereof.

The MICSSS method does not lead to antigenicity loss, steric hindrance, or increased cross-reactivity. For potentially weaker or heterogenous markers such as PD-L1, it is recommended that such antigens be stained first in any sequential staining. MICSSS implementation does not require additional instrumentation and relies on standard antigen retrieval and staining protocols, limiting the need for novel validation strategies making MICSSS a method of choice for multiplexed IHC in standard clinical pathology laboratories.

As shown in the present data, MICSSS can be used as a new tool to describe the immune microenvironment at baseline and to track immune changes upon therapy providing a unique sample sparing analytical tool to characterize limited tissue samples obtained during clinical studies. By analyzing the composition of complex immune cell populations that accumulated in the center of 75 primary NSCLC tumor lesions, a neutrophil/DC density score refined the prognostic value of tumors rich in T cells and was the best independent prognosticator (p=4.97 × 10-5), even stronger than the TNM stage (p=2.09 × 10-4). Although these findings are based upon a small number of patient samples, these data reveal MICSSS potential to expand the Immunoscore prognostic signature of human tumor lesions in a clinically relevant manner. In addition to developing a new multiplexed IHC method, a novel automated digital landscaping method has been developed to evaluate the density and spatial distribution of complex cell populations in a high-throughput manner, based on neural network marker identification and quantification on whole slides. The combination of both technologies reveals the power of multiplexed biomarker imaging and quantitative analysis for in depth tissue analysis.

In summary, these results demonstrate a novel multiplexed chromogenic IHC strategy for high dimensional tissue analysis that circumvents many of the limitations of regular chromogenic, immunofluorescence and mass cytometry approaches that could be readily implemented in clinical pathology laboratories. The MICSSS method provides a new powerful tool to map the microenvironment of tissue lesional sites with excellent resolution, in a sample-sparing manner, to monitor immune changes *in situ* during therapy and help identify novel prognostic and predictive markers of clinical outcome in patients with cancer and inflammatory diseases.

### METHODS

### Patients

Paraffin-embedded human tonsils, ulcerative colitis, NSCLC, melanoma and colorectal tumor samples were obtained from the Biorepository tissue bank at Icahn School of Medicine at Mount Sinai (ISMMS). Tissue samples were obtained according to protocols approved by the Institutional Review Board of ISMMS. Drs. Wolchok and Merghoub at Memorial Sloan Kettering Cancer Center (MSKCC) provided melanoma tumor lesions treated with ipilimumab. Patients with metastatic melanoma who were treated with ipilimumab were selected for inclusion in this analysis based upon sample availability and annotated clinical data. Clinical benefit was determined by evidence of tumor burden reduction or prolonged stable disease lasting at least 9 months following initiation of ipilimumab. Patients received ipilimumab at 3 mg/kg or 10 mg/kg as per initial study design. Two different tissue lesions were obtained from tumor excision prior and after treatment with ipilimumab and analyzed by IHC. All patients provided informed consent to an Institutional Review Board approved correlative research protocol prior to the collection of tissue (Memorial Sloan Kettering Cancer Center IRB # 00-144). TMA displaying 75 lung adenocarcinomas were purchased from US Biomax Inc. The TMA contained human tissues obtained with informed consent according to US federal law. The Reporting Recommendations for Tumor Marker Prognostic Studies (REMARK) criteria (27) were followed throughout this study.

### Immunohistochemistry

Five-microns FFPE tissue sections were deparaffinized in xylene and rehydrated in decreasing concentrations of ethanol (100%, 90%, 70%, 50% and distilled water; 5 minutes each time). Rehydrated tissue sections were incubated in pH6 or pH9 Target Retrieval Solution (Dako, S2369 and S2367) for antigen retrieval at 95°C for 30 minutes. Tissue sections were incubated in 3% hydrogen peroxide for 15 minutes to block endogenous peroxidase activity and in serum-free protein block solution (Dako, X0909) for 30 minutes to block free FcR binding sites before adding the primary Abs, listed in supplementary Table 1, followed by biotinylated secondary Abs. Binding of biotinylated Abs was revealed by streptavidin-horseradish peroxidase and chromogenic revelation was done using 3-amino-9 ethylcarbazole (AEC, Vector, SK-4200) or 3,3'- Diaminobenzidine (DAB, Dako, K3468). Nonspecific isotype controls were used as negative controls. Tissue sections were then counterstained with Harris modified hematoxylin (Sigma, HHS16), mounted with aqueous mounting medium (Dako, C0563) and scanned for digital imaging and quantification (Olympus whole-slide scanner with Olyvia software or a Nikon Eclipse Ci-E microscope). After scanning, slides coverslips were removed and tissue sections were destained in organic solvent. This step removed staining with labile AEC precipitate while leaving DAB unaffected. Then, the slides were mounted with aqueous mounting medium and stored at 4°C up to several months or directly subjected to the next round of staining as previously described with some modifications. Antigen retrieval was performed before incubating each slide with blocking solution for 30 minutes and endogenous biotin was blocked using streptavidin/biotin blocking kit (Vector, SP-2002). In the next step, the tissue sections were stained as previously described.

### Examplary Manual MICSSS Staining Protocol for formalin-fixed paraffin-embedded tissue sections.

### Day 1 - Bake Slides

### 1. Bake slides overnight at 37°C

### Day 2 - Antibody 1 Staining

### Deparaffinization and Rehydration Steps

2. Immerse slides in 100% xylene for 5 minutes, 3X each for 5 mins
   —> Gently drain excess liquid between each step
   —> Do not dry tissue once started
   —> Do steps 2 - 7 in fume hood
   —> Can reuse solutions from steps 2 - 7 up to 20 times (or until gets dirty)
3. Immerse slides in 100% ethanol for 5 mins
4. Immerse slides in 90% ethanol for 5 mins
5. Immerse slides in 70% ethanol for 5 mins
6. Immerse slides in 50% ethanol for 5 mins
7. Immerse slides in dH₂O for 5 mins

### Heat-induced Epitope Retrieval

8. Dilute 10X Target Retrieval Solution (RS) to 1X - use correct pH for antigen 1
   —> Use pH 6, pH 8 or pH 9 depending on antigen
   —> Prepare 40mL for up to 2 slides
9. Pre-heat the RS to 95°C in a water bath
10. Immerse slides in the 50mL conical of 95°C RS
   → Can add up to 2 slides in one 50mL conical, back-to-back
11. Incubate in the 95°C water bath for 30 mins
12. Remove conicals from water bath and place at RT
13. Open caps of conicals and incubate at RT for 30 mins
14. Rinse slides with Tris Buffered Saline (TBS)
15. Dry the back of the slides and around the tissue section

### → DO NOT TOUCH THE TISSUE

### Blocking

16. Cover tissue with 3% peroxidase (H₂O₂) and incubate for 15 mins
   —> This quenches endogenous peroxidase activity
   —> Generally 1-3 drops covers tissue
17. Rinse slides with TBS
18. Dry the back of the slides and around the tissue section
   → **DO NOT TOUCH THE TISSUE**
19. Cover tissue with Serum-Free Protein Block (SFPB, Dako) and incubate for 30 mins
20. Rinse slides with TBS
21. Dry the back of the slides and around the tissue section

### → DO NOT TOUCH THE TISSUE

### Primary Staining

22. Dilute primary antibody in REAL Antibody Diluent (RAD, Dako) to working concentration
23. Cover tissue with primary antibody solution and incubate for 1 hr
24. Rinse slides briefly with TBS
25. Immerse slides in TBS + 0.04% Tween 20 (TBS20) for 5 mins
26. Dry the back of the slides and around the tissue section

### → DO NOT TOUCH THE TISSUE

### Secondary Staining

27. Dilute secondary antibody in TBS to working concentration
28. Cover tissue with secondary antibody solution and incubate for 30 mins
29. Immerse slides in TBS20 for 5 mins
30. Dry the back of the slides and around the tissue section

### → DO NOT TOUCH THE TISSUE

31. Dilute streptavidin-HRP in TBS to working concentration
32. Cover tissue with HRP solution and incubate for 30 mins Steps 27 →32 can be replaced by incubation for 30 minutes with labeled Polymer-Dako REAL EnVision-HRP (anti-mouse or anti-rabbit)
33. Immerse slides in TBS20 for 5 mins
34. Immerse slides in TBS for 2 mins
35. Dry the back of the slides and around the tissue section

### → DO NOT TOUCH THE TISSUE

### Antigen Detection

36. Prepare (fresh) AEC solution (Vector)
   - > AEC is a red dye that can be bleached for multiplexing antibodies
   - > Do not use DAB as it cannot be bleached 37. Cover tissue with AEC solution and incubate for 4 - 5 mins
   - > Up to 30 mins
   - > Background staining will appear homogenously red 38. Check the intensity of the staining under a microscope
   - > Look under light microscope with solution still on slide to determine when to stop
39. Rinse slides with dH₂O for 5 mins
40. Counterstain by adding slides to 100% hematoxylin for 5-15 seconds
   → Can save the hematoxylin after use
41. Rinse slides with dH₂O
   → Rinse with lots of dH₂O (~2L)
42. Mount the slides using a coverslip in Aqueous Mounting Medium (Dako)
   —> Need to warm mounting medium before use
   —> Use ~100µL per coverslip, then add slide on top
43. Incubate slides at RT until mounting medium solidifies
44. Visualize staining using microscope

### Storage

45. Can store slides at 4°C for less than a week but the longer the AEC red dye is left, the harder it is to bleach
46. For longer term storage (months), go do Day 3 and continue on to bleaching steps 1 - 6 (Day 3) and then mount the slides like in step 42

### Day 3 - Antibody 2 Staining

### Bleaching

1. Remove coverslip by adding slide to warm/hot water
2. Immerse slides in dH₂O for 2 mins
3. Immerse slides in 50% ethanol for 2 mins
4. Immerse slides in 100% ethanol for 5 mins
5. Immerse slides in 50% ethanol for 2 mins
6. Immerse slides in dH₂O for 5 mins

An alternative in step 4 is to utilize 90% ethanol.

### Heat-induced Epitope Retrieval

7. Dilute 10X RS to 1X - use correct pH for antigen 2
   —> Use pH 6 or pH 9 depending on antigen
   —> Prepare 40mL for up to 2 slides
8. Pre-heat the RS to 95°C in a water bath
9. Immerse slides in the 50mL conical of 95°C RS
   → Can add up to 2 slides in one 50mL conical, back-to-back
10. Incubate in the 95°C water bath for 5-15 mins
11. Remove conicals from water bath and place at RT
12. Open caps of conicals and incubate at RT for 30 mins
13. Rinse slides with TBS
14. Dry the back of the slides and around the tissue section

### → DO NOT TOUCH THE TISSUE

### Blocking

15. Cover tissue with 3% peroxidase (H₂O₂) + sodium azide 1mM and incubate for 20 mins
   → This quenches endogenous peroxidase activity
   → Generally 1-3 drops covers tissue
16. Rinse slides with TBS
17. Dry the back of the slides and around the tissue section

### → DO NOT TOUCH THE TISSUE

18. Cover tissue with SFPB and incubate for 30 mins
19. Rinse slides with TBS
20. Dry the back of the slides and around the tissue section

### → DO NOT TOUCH THE TISSUE

21. Cover tissue with Avidin/Biotin Blocking Kit Avidin solution (Dako) and incubate for 30 mins
22. Immerse slides in TBS for 1 min
23. Dry the back of the slides and around the tissue section

### → DO NOT TOUCH THE TISSUE

24. Cover tissue with Avidin/Biotin Blocking Kit Avidin solution (Dako) and incubate for 30 mins
   No need to block biotin/streptavidin if Polymer- Dako REAL EnVision-HRP (anti-mouse or anti-rabbit) was used for the previous staining
25. Immerse slides in TBS for 1 min
26. Dilute Serum (final concentration: 10%) in TBS + 10% Avidin solution

### (Dako)

—> Use the **same species for the serum** (e.g. if you are using 2 rabbit primary antibodies, use rabbit serum control Ig)
   27. Cover tissue and incubate for 30 mins
   28. Immerse slides in TBS20 for 5 mins
   29. Dry the back of the slides and around the tissue section

### → DO NOT TOUCH THE TISSUE

30. Dilute FAb anti-**animal** IgG in TBS + 10% Biotin solution (Dako)
   - > Just like the serum, use FAbs that are the same species as the overlapping primary antibodies
31. Cover tissue with FAb anti-animal IgG and incubate for 30 mins
32. Immerse slides in TBS20 for 5 mins
33. Dry the back of the slides and around the tissue section

### → DO NOT TOUCH THE TISSUE

### Primary Staining

34. Dilute primary antibody 2 in REAL Antibody Diluent (RAD, Dako) to working concentration
35. Cover tissue with primary antibody solution and incubate for 1 hr
36. Rinse slides briefly with TBS
37. Immerse slides in TBS + 0.04% Tween 20 (TBS20) for 5 mins
38. Dry the back of the slides and around the tissue section

### → DO NOT TOUCH THE TISSUE

### Secondary Staining

39. Dilute secondary antibody in TBS to working concentration
40. Cover tissue with secondary antibody 2 solution and incubate for 30 mins
41. Immerse slides in TBS20 for 5 mins
42. Dry the back of the slides and around the tissue section

### → DO NOT TOUCH THE TISSUE

43. Dilute streptavidin-HRP in TBS to working concentration
44. Cover tissue with HRP solution and incubate for 30 mins
Steps 39 →44 can be replaced by incubation for 30 minutes with labeled Polymer-Dako REAL EnVision-HRP (anti-mouse or anti-rabbit)
45. Immerse slides in TBS20 for 5 mins
46. Immerse slides in TBS for 2 mins
47. Dry the back of the slides and around the tissue section

### → DO NOT TOUCH THE TISSUE

### Antigen Detection

48. Prepare (fresh) AEC solution
   → AEC is a red dye that can be bleached for multiplexing antibodies
   → Do not use DAB as it cannot be bleached
49. Cover tissue with AEC solution and incubate for 4 - 5 mins
   → Up to 30 mins
   → Background staining will appear homogenously red
50. Check the intensity of the staining under a microscope
   → Look under light microscope with solution still on slide to determine when to stop
51. Rinse slides with dH₂O for 5 mins
52. Counterstain by adding slides to 100% hematoxylin for 5-15 seconds
   → Can save the hematoxylin after use
53. Rinse slides with dH₂O
   → Rinse with lots of dH₂O (~2L)
54. Mount the slides using a coverslip in Aqueous Mounting Medium
   → Need to warm mounting medium before use
   → Use ~100µL per coverslip, then add slide on top
55. Incubate slides at RT until mounting medium solidifies
56. Visualize staining using microscope

### Day 4+ - Antibody 3+ Staining

### 57. Repeat like day 3

**TABLE 3: Primary Antibodies used for IHC**

| **Antibody** | **Species** | **Isotype** | **Clone** | **Antige n** | **Dilution** | **Subcellular localization** |
|---|---|---|---|---|---|---|
| **Anti-CCL19** | Goat | IgG | Polyclonal | Buffer pH6 | 1/80 | Cytoplasmic |
| **Anti-CD1a** | Mouse | IgG1 | O10 | BufferpH6 | 1/50 | Membranous/ cytoplasmic |
| **Anti-CD1c** | Mouse | IgG1 | 2F4 | Buffer pH9 | 1/150 | Membranous/ cytoplasmic |
| **Anti-CD2** | Mouse | IgG1 | AB75 | BufferpH9 | 1/40 | Membranous |
| **Anti-CD3** | Rabbit | IgG | 2GV6 | BufferpH9 | RTU | Membranous |
| **Anti-CD8** | Mouse | IgG1 | C8/144b | BufferpH9 | 1/100 | Membranous |
| **Anti-CD20** | Mouse | IgG2a | L26 | Buffer pH9 | 1/250 | Membranous |
| **Anti-CD66b** | Mouse | IgM | G10F5 | BufferpH9 | 1/600 | Membranous |
| **Anti-CD68** | Mouse | IgG1 | KP1 | Buffer pH6 | 1/400 | Membranous/ cytoplasmic |
| **Anti-CD138** | Mouse | IgG1 | MI15 | BufferpH9 | 1/100 | Cytoplasmic |
| **Anti-CD206** | Rabbit | IgG | Polyclonal | Buffer pH6 | 1/500 | Cytoplasmic |
| **Anti-DC-LAMP** | Rat | IgG2a | 1010E1.01 | BufferpH6 | 1/80 | Cytoplasmic |
| **Anti-Cytokeratin** | Mouse | IgG1 | AE1/AE3 | Buffer pH6 | 1/50 | Cytoplasmic |
| **Anti-Foxp3** | Mouse | IgG1 | 236A/E7 | BufferpH6 | 1/80 | Nuclear |
| **Anti-Ki-67** | Rabbit | IgG | 30-9 | BufferpH9 | 1/100 | Nuclear |
| **Anti-PD1** | Mouse | IgG1 | NAT105 | BufferpH6 | 1/50 | Membranous |
| **Anti-PD-L1** | Rabbit | IgG | E1L3N | BufferpH9 | 1/100 | Membranous |
| **Anti-HLA** | Mouse | IgG1 | TAL1B5 | BufferpH9 | 1/500 | Membranous/ cytoplasmic |
| **Anti-HLA Class I** | Mouse | IgG1 | EMR8-5 | BufferpH6 | 1/200 | Membranous |

### Exemplary Automated MICSSS Staining Protocol for formalin-fixed paraffin-embedded tissue sections.

### Day 1 - Bake Slides

### 1. Bake slides overnight at 37°C

### Day 2 - Antibody 1 Staining

2. Place the slides on racks (up to 48 slides)
   *Deparaffinization, Rehydration Steps and Heat-induced Epitope Retrieval using PT Link, Pre-Treatment Module for Tissue Specimens (Dako)*
3. Pre-heat the low or high (depending on the Ag) pH solutions at 75°C
4. Place the slides in the PT-Link
5. Start the run on the PT-link (20 mins 100°C)
6. Rinse slides with TBS for 5 mins
7. Place the slides in the Link-48 autostainer (Dako)
8. Start the staining program with the following steps:
   a. Rinse (buffer)
   b. 3% peroxidase (H₂O₂): 15 mins
   c. Rinse (buffer)
   d. Serum-Free Protein Block (SFPB, Dako): 30 mins
   e. Rinse (buffer)
   f. Primary Ab: 1 hour
   g. Rinse buffer
   h. Labeled Polymer- Dako REAL EnVision-HRP: 30 mins
   i. Rinse buffer
   j. AEC: 5-30 minutes
   k. Rinse buffer (water)
   l. Hematoxylin: 2 minutes
   m. Rinse buffer (water)
9. Mount the slides using a coverslip in Aqueous Mounting Medium (Dako)
   - > Need to warm mounting medium before use
   - > Use ~100µL per coverslip, then add slide on top
10. Incubate slides at RT until mounting medium solidifies
11. Visualize staining using microscope

### Storage

12. Can store slides at 4°C for less than a week but the longer the AEC red dye is left, the harder it is to bleach
13. For longer term storage (months), go do Day 3 and continue on to bleaching steps 1-7 and then mount the slides like in step 9.

### Day 3 - Antibody 2 Staining

### Place the slides on racks (up to 48 slides)

### Bleaching

14. Remove coverslip by placing slide to warm/hot water
15. Immerse slides in dH₂O for 2 mins
16. Immerse slides in 50% ethanol for 2 mins
17. Immerse slides in 100% ethanol for 6 mins
18. Immerse slides in 50% ethanol for 2 mins
19. Immerse slides in dH₂O for 2 mins

An alternative in step 17 is to utilize 90% ethanol.

### Heat-induced Epitope Retrieval (PT-Link, Dako)

20. Pre-heat the low or high pH solutions (depending on the Ag) at 75°C
21. Place the slides in the PT-Link
22. Start the run on the PT-link (5 mins 100°C)
23. Rinse slides with TBS for 5 mins
24. Place the slides in the Link-48 autostainer (Dako)
25. Start the staining program with the following steps:
   a. Rinse (buffer)
   b. 3% peroxidase (H₂O₂): 15 mins
   c. Rinse (buffer)
   d. Serum-Free Protein Block (SFPB, Dako): 30 mins
   e. Rinse (buffer)
   f. 10% serum: 30 mins
   g. Rinse (buffer)
   h. FAb anti-animal: 30 mins
   i. Rinse (buffer)
   j. Primary Ab: 1 hour
   k. Rinse buffer
   l. Labeled Polymer- Dako REAL EnVision-HRP: 30 mins
   m. Rinse buffer
   n. AEC: 10 minutes
   o. Rinse buffer
   p. Hematoxylin: 2 minutes
   q. Rinse buffer (water)
26. Mount the slides using a coverslip in Aqueous Mounting Medium (Dako)
   —> Need to warm mounting medium before use
   —> Use ~100µL per coverslip, then add slide on top
27. Incubate slides at RT until mounting medium solidifies
28. Visualize staining using microscope

### Day 4+ - Antibody 3+ Staining

### 29. Repeat like day 3

### Melanin bleaching

All melanoma tissue sections were incubated in 3% hydrogen peroxide + 1% Na₂HPO₄ solution for 12h at room temperature prior to incubation with primary Abs to remove the melanin granules.

### Microscopy and image analysis

Images were acquired using an Olympus whole-slide scanner with Olyvia software or a Nikon Eclipse Ci-E microscope. Each stain was artificially attributed a color code and images were overlaid using ImageJ or Adobe Photoshop CS6. Pixel colocalization was assessed by calculating Manders' overlap coefficient with threshold set by Costes method (tM) using Fiji (Coloc2 plugin). Tissue-associated immune cell densities were measured in a blinded fashion without knowledge of clinical characteristics or outcome as previously described (11) on the whole tissue (for the TMAs) or on the three most infiltrated fields28 and validated using CellProfiler 2.1.1 (Broad Institute) (29). Significant correlation was found between manual and automatic quantifications (r=0.99 and p<0.0001 (Spearman test). Immune cell density was expressed as an absolute number of positive cells/mm2. The density of MHC Class I+ cells was also assessed semiquantitatively as 1 (<25% of positive cells), 2 (25-50%), 3 (51-75%) or 4 (>75%). The density of Ki-67 positive immune (KI-67-I) or Ki-67 positive tumor (Ki-67-T) cells was assessed semi-quantitatively as low (≤10%) or high (>10%) density.

### Automated image analysis

### Spatial alignment of whole-slide images

The whole-slide image of the Ki-67-stained TMA was spatially aligned to the CD3-stained whole-slide image. The images were first roughly aligned using a template matching technique at low resolution (64 times down sampled). This resulted in an initial translation vector and rotation angle to map the positions in the CD3-stained slide to the Ki-67-stained slide. Subsequently, the elastix toolbox was used to obtain the affine transformation to minimize the differences between the images (using normalized mutual information as a metric) (30, 31). The resultant combined transformation can be used to, for each position in the CD3-stained slide, obtain the corresponding position in the Ki-67- stained slide. This approach does not address small, local deformations, but these are expected to be minimal due to the careful multiplexing procedure.

### Positive cell detection using convolutional neural networks

In each stained slide, positive cells where identified through the use of convolutional neural networks, used mainly in generic computer vision tasks32. Our approach is similar to the one presented by Ciresan *et al.* for the detection of mitosis in hematoxylin-eosin stained images of breast cancer33. First, an observer (G.L.) annotated 3500 positive nuclei across all TMA spots and indicated regions containing normal tissue and tar to serve as the negative class. Subsequently, 45x45 pixel patches were sampled from the positive nuclei and the background regions to train a five-layer convolutional network. This network was then used to estimate the posterior likelihood of being part of a positive nucleus for each pixel in the CD3 and Ki-67 whole slide images. To prevent any bias in the results this training/classification step was performed in a two-fold cross validation, where half the TMA spots served as training data and half were classified.

### Post-processing steps and cell counting

To extract the center pixels for each nucleus, we applied a fast radial symmetry transform (FRST) approach to the generated likelihood maps (34). This step helps remove false positive in dense cell clusters by focusing only on radially symmetric objects (e.g. cell nuclei) and identifies their center pixels. The registration transformation, for each positive pixel in the CD3-stained image helps assessed additional positive pixel in the Ki-67-stained image. These data resulted in three images, one containing all the pixels that were CD3-positive, one containing all the pixels that were Ki-67-positive and one containing all the double-positive pixels. Connected component analysis was subsequently applied to extract the total number of positive cells for each of these images. Thus, for each TMA-spot, the total number of CD3-positive, Ki-67-positive and double-positive cells was obtained.

### Statistical analysis

Sample size calculation for the prognostic biomarker analysis was performed using the method described by Schoenfeld *et al*.35. For each biomarker, the proportions of subjects in low and high groups were based on published studies reviewed by Remark *et al.23.* Associations of variables to prognosis were visualized using the Kaplan-Meier method and significant differences of overall survival among patient groups were calculated with the log-rank test. The following cutoffs were used to discriminate low and high groups for the survival analyses using the "minimum p value approach"6: 130.3 cells/mm2 (CD68), 9.8 cells/mm2 (CD66b), 0.42 cells/mm2 (DC-LAMP), 1.13 cells/mm2 (CD1c), 1.27 cells/mm2 (CD20), 59.1 cells/mm2 (CD3), 7.5 cells/mm2 (FoxP3), 25% (Ki- 7-T) and 10% (Ki-67-I). To avoid over-fitting, we corrected overall survival log-rank p values obtained by the "minimum p value" approach, as previously reported (6). Multivariate Cox proportional hazards were used model to determine hazard ratios. To be able to conduct regression with categorical variables, each variable was coded before being entered into the Cox model. Proportional hazard assumption (PHA) was assessed and respected for each variable. The nonparametric Mann-Whitney test was used to compare the density of infiltrating immune cells between different groups of patients and correlations were evaluated by the nonparametric Spearman test. All p values were calculated using two-sided tests. P values <0.05 were considered statistically significant. Analyses were performed using GraphPad Prism version 6.00 (GraphPad Software, La Jolla California USA) and R version 3.1.3 (http://www.r-project.org/).

### REFERENCES

1. De Obaldia ME, Bhandoola A. Transcriptional regulation of innate and adaptive lymphocyte lineages. Annual review of immunology. 2015;33:607-642.
2. Spits H, Cupedo T. Innate lymphoid cells: emerging insights in development, lineage relationships, and function. Annual review of immunology. 2012;30:647-675.
3. van de Pavert SA, Mebius RE. New insights into the development of lymphoid tissues. Nature reviews. Immunology. 2010;10(9):664-674.
4. Fridman WH, Pages F, Sautes-Fridman C, Galon J. The immune contexture in human tumours: impact on clinical outcome. Nature reviews. Cancer. 2012;12(4):298-306.
5. Shalapour S, Karin M. Immunity, inflammation, and cancer: an eternal fight between good and evil. The Journal of clinical investigation. 2015;125(9):3347-3355.
6. Galon J, Costes A, Sanchez-Cabo F, et al. Type, density, and location of immune cells within human colorectal tumors predict clinical outcome. Science. 2006;313(5795):1960-1964.
7. Fridman WH, Remark R, Goc J, et al. The immune microenvironment: a major player in human cancers. International archives of allergy and immunology. 2014;164(1):13-26.
8. Galon J, Angell HK, Bedognetti D, Marincola FM. The continuum of cancer immunosurveillance: prognostic, predictive, and mechanistic signatures. Immunity. 2013;39(1):11-26.
9. Galon J, Pages F, Marincola FM, et al. Cancer classification using the Immunoscore: a worldwide task force. Journal of translational medicine. 2012;10:205.
10. Goc J, Germain C, Vo-Bourgais TK, et al. Dendritic Cells in Tumor-Associated Tertiary Lymphoid Structures Signal a Th1 Cytotoxic Immune Contexture and License the Good Positive Prognostic Value of Infiltrating CD8+ T Cells. Cancer research. 2014.
11. Remark R, Alifano M, Cremer I, et al. Characteristics and clinical impacts of the immune environments in colorectal and renal cell carcinoma lung metastases: influence of tumor origin. Clinical cancer research : an official journal of the American Association for Cancer Research. 2013;19(15):4079-4091.
12. Remark R, Becker C, Gomez JE, et al. The Non-Small Cell Lung Cancer Immune Contexture: a Major Determinant of Tumor Characteristics and Patient Outcome. Am J Respir Crit Care Med. 2014.
13. Pardoll DM. The blockade of immune checkpoints in cancer immunotherapy. Nature reviews. Cancer. 2012;12(4):252-264.
14. Page DB, Postow MA, Callahan MK, Allison JP, Wolchok JD. Immune modulation in cancer with antibodies. Annual review of medicine. 2014;65:185-202.
15. Herbst RS, Soria JC, Kowanetz M, et al. Predictive correlates of response to the anti-PD-L1 antibody MPDL3280A in cancer patients. Nature. 2014;515(7528):563-567.
16. Tumeh PC, Harview CL, Yearley JH, et al. PD-1 blockade induces responses by inhibiting adaptive immune resistance. Nature. 2014;515(7528):568-571.
17. Wolchok JD, Chan TA. Cancer: Antitumour immunity gets a boost. Nature. 2014;515(7528):496-498.
18. Robertson D, Savage K, Reis-Filho JS, Isacke CM. Multiple immunofluorescence labelling of formalin-fixed paraffin-embedded (FFPE) tissue. BMC cell biology. 2008;9:13.
19. Giesen C, Wang HA, Schapiro D, et al. Highly multiplexed imaging of tumor tissues with subcellular resolution by mass cytometry. Nature Methods. 2014;11(4):417-422.
20. Gerdes MJ, Sevinsky CJ, Sood A, et al. Highly multiplexed single-cell analysis of formalin-fixed, paraffin-embedded cancer tissue. Proceedings of the National Academy of Sciences of the United States of America. 2013;110(29):11982-11987.
21. Stack EC, Wang C, Roman KA, Hoyt CC. Multiplexed immunohistochemistry, imaging, and quantitation: a review, with an assessment of Tyramide signal amplification, multispectral imaging and multiplex analysis. Methods. 2014;70(1):46-58.
22. Angelo M, Bendall SC, Finck R, et al. Multiplexed ion beam imaging of human breast tumors. Nature medicine. 2014;20(4):436-442.
23. Remark R, Becker C, Gomez JE, et al. The non-small cell lung cancer immune contexture. A major determinant of tumor characteristics and patient outcome. American journal of respiratory and critical care medicine. 2015;191(4):377-390.
24. Lesokhin AM, Callahan MK, Postow MA, Wolchok JD. On being less tolerant: enhanced cancer immunosurveillance enabled by targeting checkpoints and agonists of T cell activation. Science translational medicine. 2015;7(280):280sr281.
25. Postow MA, Chesney J, Pavlick AC, et al. Nivolumab and ipilimumab versus ipilimumab in untreated melanoma. The New England journal of medicine. 2015;372(21):2006-2017.
26. Sharma P, Allison JP. The future of immune checkpoint therapy. Science. 2015;348(6230):56-61.
27. McShane LM, Altman DG, Sauerbrei W, et al. Reporting recommendations for tumor marker prognostic studies. Journal of clinical oncology: official journal of the American Society of Clinical Oncology. 2005;23(36):9067-9072.
28. Anitei MG, Zeitoun G, Mlecnik B, et al. Prognostic and predictive values of the immunoscore in patients with rectal cancer. Clinical cancer research: an official journal of the American Association for Cancer Research. 2014;20(7):1891-1899.
29. Jones TR, Kang IH, Wheeler DB, et al. CellProfiler Analyst: data exploration and analysis software for complex image-based screens. BMC bioinformatics. 2008;9:482.
30. Klein S, Staring M, Murphy K, Viergever MA, Pluim JP. elastix: a toolbox for intensity-based medical image registration. IEEE transactions on medical imaging. 2010;29(1): 196-205.
31. Mueller D, Vossen D, Hulsken B. Real-time deformable registration of multimodal whole slides for digital pathology. Computerized medical imaging and graphics: the official journal of the Computerized Medical Imaging Society. 2011;35(7-8):542-556.
32. LeCun Y, Bengio Y, Hinton G. Deep learning. Nature. 2015;521(7553):436-444.
33. Ciresan DC, Giusti A, Gambardella LM, Schmidhuber J. Mitosis detection in breast cancer histology images with deep neural networks. Medical image computing and computer-assisted intervention : MICCAI ... International Conference on Medical Image Computing and Computer-Assisted Intervention. 2013;16(Pt 2):411-418.
34. Loy G, Zelinsky A. Fast radial symmetry for detecting points of interest. IEEE transactions on pattern analysis and machine intelligence. 2003;25(8):959-973.
35. Schoenfeld DA. Sample-size formula for the proportional-hazards regression model. Biometrics. 1983;39(2):499-503.

## Claims

1. A method of detecting multiple antigens from a formalin-fixed paraffin-embedded tissue sample comprising:
(a) subjecting the sample to an antigen retrieval process to expose one or more antigens in the sample, wherein the antigen retrieval process is performed at a specific pH6 or pH9 for the antigen being retrieved;
(b) applying a hydrogen peroxide to block endogenous detection of peroxidase
(c) applying a second blocking reagent to block against nonspecific binding of one or more antigens;
(d) incubating the sample with an antibody that binds to one target in the sample;
(e) incubating the sample with a secondary antibody bound to HRP;
(f) incubating the sample with 3-amino-9-ethylcarbazole (AEC);
(g) detecting a signal from the AEC;
(h) removing the AEC or destaining the signal from step (d) by sequentially:
immersing the sample in an organic solvent-based destaining buffer comprising 50% ethanol for 2 mins,
immersing the sample in an organic solvent-based destaining buffer comprising 90% ethanol for 5 mins, and
immersing the sample in an organic solvent-based destaining buffer comprising 50% ethanol for 2 mins, wherein the removal of AEC or destaining does not remove the antibody and allows for the preservation of tissue antigenicity and tissue architecture of the sample and the repeated performance of every step on the sample;
(i) repeating steps (a) through (h) at least one time, and wherein when steps (a) through
(h) are repeated, the method comprises a further step of applying a third blocking reagent to block against specific binding of the antibody used previously in step (d).

2. The method of claim 1, wherein the biological sample is prepared and fixed on a slide.

3. The method of claim 2, wherein the biological sample comprises frozen tissue.

4. The method of claim 2, wherein the sample is preserved for at least 6 months.

5. The method of any of claims 1 to 4, wherein steps (a) through (h) are repeated for at least 5 cycles.

6. The method of any of claims 1 to 5, wherein steps (a) through (h) are repeated for at least 10 cycles.

7. The method of any of claims 1 to 6, wherein the biological sample has previously been analyzed by *in situ* hybridization (FISH or CISH).

8. The method of any of claims 1 to 7, further comprising detecting a stained fixed biomarker in the biological sample.

9. The method of claim 8, wherein the fixed biomarker is stained with 3,3'-Diaminobenzidine: (DAB).

## Patentansprüche

1. Ein Verfahren zum Detektieren multipler Antigene aus einer Formalinfixierten, in Paraffin eingebetteten Gewebeprobe, wobei das Verfahren Folgendes aufweist:
(a) Unterziehen der Probe einem Antigen-Rückgewinnungsprozess, um ein oder mehrere Antigene in der Probe zu exponieren, wobei der Antigen-Rückgewinnungsprozess bei einem spezifischen pH6 oder pH9 für das rückzugewinnende Antigen durchgeführt wird;
(b) Aufbringen eines Wasserstoffperoxids, um endogene Detektion von Peroxidase zu blockieren;
(c) Aufbringen eines zweiten Blockier-Reagens, um gegen unspezifisches Binden eines oder mehrerer Antigene zu blockieren;
(d) Inkubieren der Probe mit einem Antikörper, der an ein Target in der Probe bindet;
(e) Inkubieren der Probe mit einem an HRP gebundenen sekundären Antikörper;
(f) Inkubieren der Probe mit 3-Amino-9-Ethylcarbazol (AEC);
(g) Detektieren eines Signals vom AEC;
(h) Entfernen des AEC oder Entfärben des Signals aus Schritt (d) durch sequenzielles:
Eintauchen der Probe in einen auf organischem Lösungsmittel basierenden Entfärbungspuffer, der 50 % Ethanol aufweist, für 2 min,
Eintauchen der Probe in einen auf organischem Lösungsmittel basierenden Entfärbungspuffer, der 90 % Ethanol aufweist, für 5 Minuten, und
Eintauchen der Probe in einen auf organischem Lösungsmittel basierenden Entfärbungspuffer, der 50 % Ethanol aufweist, für 2 Minuten, wobei die Entfernung von AEC oder Entfärben den Antikörper nicht entfernt und die Erhaltung von Gewebe-Antigenität und von Gewebe-Architektur der Probe und die wiederholte Durchführung jedes Schritts an der Probe ermöglicht;
(i) Wiederholen der Schritte (a) bis (h) mindestens einmal, und wobei, wenn die Schritte (a) bis (h) wiederholt werden, das Verfahren einen weiteren Schritt von Aufbringen eines dritten Blockier-Reagens zum Blockieren gegen spezifisches Binden des zuvor in Schritt (d) verwendeten Antikörpers aufweist.

2. Das Verfahren nach Anspruch 1, wobei die biologische Probe auf einem Objektträger vorbereitet und fixiert ist.

3. Das Verfahren nach Anspruch 2, wobei die biologische Probe gefrorenes Gewebe aufweist.

4. Das Verfahren nach Anspruch 2, wobei die Probe für mindestens 6 Monate konserviert wird.

5. Das Verfahren nach einem der Ansprüche 1 bis 4, wobei Schritte (a) bis (h) für mindestens 5 Zyklen wiederholt werden.

6. Das Verfahren nach einem der Ansprüche 1 bis 5, wobei Schritte (a) bis (h) für mindestens 10 Zyklen wiederholt werden.

7. Das Verfahren nach einem der Ansprüche 1 bis 6, wobei die biologische Probe zuvor durch *in situ* Hybridisierung (FISH oder CISH) analysiert worden ist.

8. Das Verfahren nach einem der Ansprüche 1 bis 7, das ferner Detektieren eines angefärbten fixierten Biomarkers in der biologischen Probe aufweist.

9. Das Verfahren nach Anspruch 8, wobei der fixierte Biomarker mit 3,3-Diaminobenzidin (DAB) angefärbt ist.

## Revendications

1. Méthode de détection de multiples antigènes à partir d'un échantillon de tissu enrobé dans la paraffine et fixé au formol comprenant :
(a) la soumission de l'échantillon à un procédé de récupération d'antigène pour exposer un ou plusieurs antigènes dans l'échantillon, dans laquelle le procédé de récupération d'antigène est réalisé à un pH spécifique de 6 ou de 9 pour que l'antigène puisse être récupéré ;
(b) l'application de peroxyde d'hydrogène pour bloquer la détection endogène de peroxydase
(c) l'application d'un deuxième réactif de blocage pour bloquer la liaison non spécifique d'un ou de plusieurs antigènes ;
(d) la mise en incubation de l'échantillon avec un anticorps qui se lie à une cible dans l'échantillon ;
(e) la mise en incubation de l'échantillon avec un anticorps secondaire lié à la HRP ;
(f) la mise en incubation de l'échantillon avec le 3-amino-9-éthylcarbazole (AEC) ;
(g) la détection d'un signal provenant de l'AEC ;
(h) l'élimination de l'AEC ou la décoloration du signal provenant de l'étape (d) par séquentiellement :
l'immersion de l'échantillon dans un tampon décolorant à base de solvant organique comprenant 50 % d'éthanol pendant 2 minutes,
l'immersion de l'échantillon dans un tampon décolorant à base de solvant organique comprenant 90 % d'éthanol pendant 5 minutes, et
l'immersion de l'échantillon dans un tampon décolorant à base de solvant organique comprenant 50 % d'éthanol pendant 2 minutes, dans laquelle l'élimination de l'AEC ou la décoloration n'élimine pas l'anticorps et permet la préservation de l'antigénicité tissulaire et de l'architecture tissulaire de l'échantillon et la performance répétée de chaque étape sur l'échantillon ;
(i) la répétition des étapes (a) à (h) au moins une fois, et dans laquelle quand les étapes (a) à (h) sont répétées, la méthode comprend une étape supplémentaire d'application d'un troisième réactif de blocage pour bloquer la liaison spécifique de l'anticorps utilisé préalablement dans l'étape (d).

2. Méthode selon la revendication 1, dans laquelle l'échantillon biologique est préparé et fixé sur une lame.

3. Méthode selon la revendication 2, dans laquelle l'échantillon biologique comprend un tissu congelé.

4. Méthode selon la revendication 2, dans laquelle l'échantillon est conservé pendant au moins 6 mois.

5. Méthode selon l'une quelconque des revendications 1 à 4, dans laquelle les étapes (a) à (h) sont répétées pendant au moins 5 cycles.

6. Méthode selon l'une quelconque des revendications 1 à 5, dans laquelle les étapes (a) à (h) sont répétées pendant au moins 10 cycles.

7. Méthode selon l'une quelconque des revendications 1 à 6, dans laquelle l'échantillon biologique a été préalablement analysé par hybridation *in situ* (FISH ou CISH).

8. Méthode selon l'une quelconque des revendications 1 à 7, comprenant en outre la détection d'un biomarqueur fixé coloré dans l'échantillon biologique.

9. Méthode selon la revendication 8, dans laquelle le biomarqueur fixé est coloré avec la 3,3'-diamino-benzidine : (DAB).
